(19) Europäisches Patentamt
European Patent Office
Office européen des brevets

(11) EP 3 017 809 A1

# (12) EUROPEAN PATENT APPLICATION

(43) Date of publication:
**11.05.2016 Bulletin 2016/19**

(21) Application number: **14192372.2**

(22) Date of filing: **07.11.2014**

(51) Int Cl.:
***A61K 9/00*** *(2006.01)*
***A61K 47/34*** *(2006.01)*
***A61K 31/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Ferring B.V.**
**2132 JX Hoofddorp (NL)**

(72) Inventors:
- **Halliday, Janet**
  **East Kilbride, G74 5PB (GB)**
- **Carr, Denis**
  **East Kilbride, G74 5PB (GB)**
- **Ross, Alistair**
  **East Kilbride, G74 5PB (GB)**

(74) Representative: **Gibbs, Richard**
**Marks & Clerk LLP**
**Aurora**
**120 Bothwell Street**
**Glasgow G2 7JS (GB)**

## (54) Drug-device unit containing quinagolide

(57) The present invention relates to a polymeric drug-device unit comprising a polyurethane block copolymer and an active agent. The polyurethane block copolymer is obtainable by reacting together a poly(alkylene oxide), a difunctional compound, a difunctional isocyanate and, optionally a block copolymer comprising poly(alkylene oxide) blocks. The polymeric drug-device unit generally takes the form of a vaginal ring.

EP 3 017 809 A1

## Description

### FIELD OF THE INVENTION

The present invention relates to a solid controlled release polymeric drug-device unit formed of a polyurethane block copolymer and comprising the dopamine-agonist quinagolide, particularly in the form of a vaginal ring intended for sustained release of drug to a patient. The polymeric drug-device unit is particularly, though not exclusively, intended for the treatment of endometriosis.

### BACKGROUND

Endometriosis is an estrogen-dependent chronic gynaecological disease pathologically characterized by the presence of endometrial-like tissue outside the uterus principally located on the peritoneum which lines the abdominal cavity, ovaries, and rectovaginal septum. Endometriosis is a major contributor to pelvic pain and decreased fertility Endometrial-like cells in areas outside the uterus (endometriosis) are influenced by hormonal changes and respond in a way that is similar to the cells found inside the uterus, resulting in an inflammatory response accompanied by angiogenesis, adhesions, fibrosis, scarring, neuronal infiltration, and anatomical distortion. Endometriosis is associated with various distressing symptoms including dysmenorrhoea, dyspareunia, pelvic pain and infertility. In a sub-population of patients endometriosis may develop into an aggressive, debilitating disease.

There is no cure for endometriosis but in many women it is abated after menopause. In patients in the reproductive years, endometriosis is merely managed and involves repeated courses of medical therapy, surgical therapy, or both. The goal is to provide pain relief, to restrict progression of the disease, and to restore or preserve fertility where needed. Medical therapies for endometriosis include: treatment with progesterone or progestins, hormonal contraception therapy, suppressive steroids with androgenic activity, such as danazol and gesterone, gonadotropin releasing hormone agonist treatment, and aromatase inhibitors that block the formation of estrogen. These medical therapies all have an anti-ovulatory effect which negatively impacts on fecundity.

The use of quinagolide for the treatment of endometriosis is suggested in patents US2012/0157489, US2010/0113499 and US2008/0293693. In particular, US2012/0157489 proposes the treatment of endometriosis by the delivery of quinagolide by vaginal administration, for example by vaginal pessary or tablet, or by vaginal ring.

Quinagolide is a dopamine receptor $D_2$ agonist that is used for the treatment of elevated levels of prolactin. It is commercially available under the trade name NORPROLAC® and is manufactured and used in the form of the hydrochloride salt. Quinagolide has the formula:

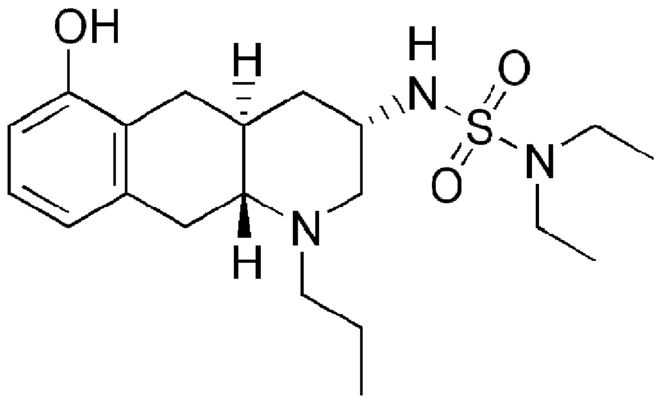

and is *N,N*-diethyl-*N'*-[(3*S*,4a*S*,10a*R*)-6-hydroxy-1-propyl-1,2,3,4,4a,5,10,10a-octahydrobenzo[*g*]quinolin-3-yl]sulfamide. The use of quinagolide to treat hyperprolactinaemia is disclosed in Eur. J. Endocrinol February 1, 2006 154 page 187-195.

The use of vaginal rings to administer drugs in a sustained release manner is known from a number of prior references. WO2009/094573 discloses the use of polyurethane vaginal rings for the delivery of progesterone. Other publications disclose the use of vaginal polyurethane rings for the sustained delivery of various drugs, including WO2010/019226, WO2004/096151, US4,235,988 and WO2005/004837, WO2013/013172. Controlled Therapeutics prior patent publication WO2008/007046 discloses the use of hydrophilic thermoplastic polyurethane elastomer polymers which are suitable for the production of controlled release compositions for the release of pharmaceutically active agents over a prolonged period of time; and particularly their use in pessaries, suppositories or vaginal rings. The use of polyether urethane elastomers for the delivery of anti-HIV drugs is also disclosed in M.R. Clark et al, Journal of Pharmaceutical

Sciences, Vol. 101, No. 2, February 2012 and also WO2012/066000.

SUMMARY OF THE INVENTION

The present invention is based on the identification of a cohort of polyurethane block copolymers that are particularly suited for use in pharmaceutical polymeric drug-device units and which offer improved control of drug release. Specifically, the polymers facilitate the control and manipulation of both the initial and sustained release kinetics and/or characteristics/properties. When loaded with quinagolide, the identified polyurethane block copolymers have been found to offer better control over its initial and long term release. The present invention further resides in the surprising discovery that quinagolide delivered in a sustained release manner using a vaginal ring formed of (or comprising) the polymers disclosed herein, is effective in the treatment and/or prevention of the chronic condition, endometriosis.

The present invention provides pharmaceutical controlled release polymeric drug-device units comprising, loaded with or having dispersed therein, quinagolide. Specifically, the polymeric drug-device units of this invention are for the controlled and/or sustained delivery of quinagolide and are formed, designed and adapted to be used, administered or worn intravaginally.

The polymeric drug-device units of this invention find particular application in the treatment and/or prevention of endometriosis. It should be understood that the terms "treatment" and "prevention" embrace any reduction or ablation of symptoms experienced by women suffering from endometriosis as well as any general inhibition and/or or slowing of the progression/development of endometriosis.

According to a first aspect, there is provided a polymeric drug-device unit comprising:

(i) a polyurethane block copolymer obtainable by reacting together:

- (a) a poly(alkylene oxide);
- (b) a difunctional compound;
- (c) a difunctional isocyanate; and
- (d) optionally a block copolymer comprising poly(alkylene oxide) blocks; and

(ii) quinagolide as a pharmaceutically active agent.

In view of the above, a polymeric drug-device unit of this invention may comprise a polyurethane block copolymer and an active agent (for example quinagolide), which active agent is contained or dispersed within the polymer. Thus the drug-device units of this invention are to be construed as devices which contain drug or active ingredient/agent. Rather, the drug-device units of this invention function actively as controlled releasing drug-carriers or devices. The term "drug-device unit" is intended to mean a combination product of drug and device/carrier (where the device or carrier may act actively or passively) by virtue of its design, physical characteristics and/or formulation properties, allow release the drug in a controlled fashion. A drug-device unit of this invention is an integrated unit which may comprise a drug (active agent) loaded polymeric system or a drug (active agent) loaded polymeric device which, in use is capable of dispensing and/or eluting an active agent. The drug-device units of this invention may be for the controlled and/or sustained delivery of an active agent.

As described in more detail below, the drug-device units of this invention may take the form of drug (active agent) loaded polymeric rings and/or pessaries for intravaginal use.

Quinagolide ($C_{20}H_{33}N_3O_3S$) is a selective, $D_2$ receptor agonist with a molecular mass of about 395 g/mol. The present invention concerns drug-device units which are loaded with quinagolide or which have quinagolide dispersed therein. Quinagolide is available under the trade name Norprolac® and as used herein, the term "quinagolide" includes all commercially available forms as well as functional derivatives and variants thereof. The term "quinagolide" also embraces all pharmaceutically acceptable (and active) salts and esters, including, for example, quinagolide hydrochloride. The term "quinagolide" also embraces any identified active enantiomers.

As such, a polymeric drug-device unit of this invention may comprise a polyurethane block copolymer as described above and a quantity of quinagolide hydrochloride loaded or dispersed therein.

It should be noted that throughout this specification the term "comprising" is used to denote that embodiments of the invention "comprise" the noted features and as such, may also include other features. However, in the context of this invention, the term "comprising" may also encompass embodiments in which the invention "consists essentially of" the relevant features or "consists of" the relevant features.

Component (a) may comprise one or more poly(alkylene oxide)s. Poly(alkylene oxide)s contain the repeating ether linkage -R-O-R- and can have two or more hydroxyl groups as terminal functional groups. They can be manufactured by the catalysed addition of cyclic ethers to an initiator in an anionic ring-opening polymerisation reaction. For example, cyclic ethers, such as ethylene oxide and propylene oxide, react with active hydrogen-containing compounds (initiators),

such as water, glycols, polyols and amines. In some cases, a catalyst may be used. For example, potassium hydroxide or sodium hydroxide are often employed as basic catalysts. After the desired degree of polymerisation has been achieved, the catalyst can be neutralized, removed by filtration and additives such as antioxidants can be added.

A wide variety of useful polyurethane block copolymers of varying structures, chain lengths and molecular weights can be made. For example, by selecting the oxide or oxides, initiator, and reaction conditions and catalysts, it is possible to polymerise a series of polyether polyols that range from low-molecular-weight poly(alkylene oxide)s to high-molecular-weight polymers. These polymers are also referred to as polyalkylene glycols or polyglycols.

In the polyurethane block copolymers employed in the present invention, the poly(alkylene oxide) may be a polyethylene glycol (PEG), a polypropylene glycol (PPG), a poly(tetramethylene oxide) (PTMO) or poly(hexamethylene oxide) (PHMO). The poly(alkylene oxide) may be polypropylene glycol.

Polyethylene glycols contain the repeat unit ($CH_2CH_2O$) and can have the structure $HO(CH_2CH_2O)_nH$ wherein n is an integer of varying size depending on the molecular weight of the polyethylene glycol. Polyethylene glycols used in the present invention are generally linear polyethylene glycols and/or generally have a molecular weight of 200 to 35,000 g/mol, particularly 1,000 to 10,000 g/mol and especially 1,500 to 5,000 g/mol. For example, the polyethylene glycol may have a molecular weight of approximately 2,000 g/mol.

Polypropylene glycols contain the repeat unit ($CH_2CH(CH_3)O$) and can have the structure $HO(CH_2CH(CH_3)O)_nH$, wherein n is an integer of varying size depending on the molecular weight of the polypropylene glycol. Polypropylene glycols used in the present invention are generally linear polypropylene glycols and/or generally have an molecular weight of 200 to 35,000 g/mol, particularly 1,000 to 10,000 g/mol and especially 1,500 to 5,000 g/mol. For example, the polypropylene glycol may have a molecular weight of approximately 2,000 g/mol.

Polypropylene glycol has unique physical and chemical properties due to the co-occurance of both primary and secondary hydroxyl groups during polymerisation, and to the multiplicity of methyl side chains on the polymers. Conventional polymerisation of propylene glycol results in an atactic polymer. The isotactic polymers mainly exist in the laboratory. Mixtures of atactic and isotactic polymers may also occur. Polypropylene has many properties in common with polyethylene glycol. Polypropylene glycols of all molecular weights are generally clear, viscous liquids with a low pour point, and which show an inverse temperature-solubility relationship, along with a rapid decrease in water solubility as the molecular weight increases. The terminal hydroxyl groups undergo the typical reactions of primary and secondary alcohols. The secondary hydroxyl group of polypropylene glycols is not as reactive as the primary hydroxyl group in polyethylene glycols.

Polyurethane block copolymers used in the present invention may be obtainable by also reacting a block copolymer comprising a poly(alkylene oxide) block together with the components (a), (b) and (c). The block copolymer comprising a poly(alkylene oxide) block may be a poly(alkylene oxide) block copolymer. The block copolymer may comprise blocks of polyethylene glycol, polypropylene glycol, a poly(tetramethylene oxide) (PTMO), poly(hexamethylene oxide) (PHMO), and/or polysiloxanes, such as polydimethylsiloxane (PDMS). The block copolymer may comprise blocks of polyethylene glycol and polypropylene glycol.

The production of block copolymers based on propylene oxide and ethylene oxide, can be initiated with ethylene glycol, glycerine, trimethylolethane, trimethylolpropane, pentaerythritol, sorbitol, sucrose and several other compounds. Mixed and alternating block copolymers can also be produced. When the secondary hydroxyl groups of PPG are capped with ethylene oxides, block copolymers of PEG and PPG with terminal primary hydroxyl groups are yielded. The primary hydroxyl groups are more reactive with isocyanates than secondary hydroxyl groups. PEG-PPG-PEG and PPG-PEG-PPG copolymers used in the present invention are generally linear having molecular weights in the range 200 to 14,000 g/mol. For example, the PEG-PPG-PEG and PPG-PEG-PPG block copolymers used in the present invention may have a molecular weight of approximately 2,000 g/mol.

As will be appreciated, the PEG content in the block copolymer may be varied. For example, a PEG-PPG-PEG copolymer may be used that comprises approximately 10% by weight of PEG. In other examples, a PPG-PEG-PPG copolymer may be used that comprises approximately 50% by weight of PEG. These exemplary block copolymers are typically commercially available. However, it will be appreciated that block copolymers having alternative compositional ranges may be used to provide pharmaceutical delivery devices according to the invention.

In this description the term "equivalent weight" is used as meaning the number average molecular weight divided by the functionality of the compound.

Component (b) may comprise one or more difunctional compound(s). The difunctional compound is reactive with the difunctional isocyanate. Suitable difunctional compounds include, for example, diols, diamines and amino alcohols.

Generally, a short chain diol is used as the difunctional compound. For example, diols in the range $C_3$ to $C_{20}$, particularly $C_4$ to $C_{10}$, especially $C_4$ to $C_6$ may be used. The diol may be a saturated or unsaturated diol. Branched or straight chain diols may be used. Representative examples of suitable diols include (but are not limited to) 1,4-butanediol, 1,5-pentanediol, 1,6-hexanediol, 1,10-decanediol, 1,12-dodecanediol and 1,16-hexadecanediol. In some cases, the use of a lower melting difunctional compound, such as pentanediol (e.g. 1,5-pentanediol), may increase the ease of manufacture of the polymer. For example, the use of pentanediol may be particularly useful when manufacturing the polymer via a reactive extrusion process.

Component (c) may comprise one or more difunctional isocyanate(s).The difunctional isocyanate may be an aromatic diisocyanate, such as diphenylmethane-4,4'-diisocyanate. The difunctional ioscyanate may be an aliphatic diisocyanate, such as dicyclohexylmethane-4,4'-diisocyanate (DMDI), hexamethylene diisocyanate (HMDI) etc. In one embodiment, the difunctional isocyanate is DMDI.

In general, the combined molar ratio of starting components (a), (b) and (d) should equal the molar ratio of starting component (c). Adhering to this general principle may ensure a balanced stoichiometry and facilitate complete (or substantially complete) reaction of all the starting polymer components. During the reaction of the starting components, one or more reaction parameters may be monitored to assess the stoichiometry and/or progress of the reaction/consumption of the starting components. The molar ratio of the components (a) to (b) to (c) is generally in the range 0.05-0.75 to 1 to 1.00-2.00. In those cases where component (d) is also present, the ratio of components (a) to (b) to (c) to (d) is generally in the range 0.05-0.75 to 1 to 1.00-2.00 to 0.01-0.50. In some cases, the molar ratio may be in the range 0.05-0.25 to 1 to 1.05-1.5 to 0.025-0.30. In other cases, the molar ratio of components may be in the range 0.05-0.20 to 1 to 1.1-1.4 to 0.03-0.25. For example, the molar ratio of components may be approximately 0.16 to 1 to 1.21 to 0.06.

As will be appreciated by the skilled person, the above molar ratios of components are based on components (a) and (d) having idealised molecular weights. By way of example, where component (a) is PPG and/or component (b) is a PPG-PEG-PPG block copolymer, the above molar ratios apply to each of those components having an idealised molecular weight of 2000. Accordingly, to ensure the weight percentages of components (a), (b), (c) and optionally (d) remain consistent in the polyurethane block copolymer when using different raw materials, the skilled person may adjust the molar ratio as appropriate (e.g. after ascertaining the exact average molecular weight of components (a) and (d)).

The polymers of this invention are generally produced by reacting one or more of the components (for example components (a), (b) and (c) above)) together in the presence of a catalyst. Suitable catalysts may include for example, tin-free catalysts based on metal carboxylates, e.g. bismuth or zinc alkanoate catalysts. Such catalysts may be based on the complexation of bismuth or zinc with organic acids comprising from 2 to 12 carbon atoms and which may be branched or straight chained, e.g. bismuth neodecanoate. Representative examples may include, but are not limited to, catalysts sold under the tradename BiCat® or Borchi® Kat. Other catalysts that may be used include, for example, ferric chloride, bismuth chloride, zinc chloride and aluminium chloride. Alternatively, tin-based catalysts (such as stannous octoate), and/or amine-based catalysts (e.g, triethylenediamine (TEDA) or 1,4-diazabicyclo[2.2.2]octane (DABCO)) may be used.

The catalyst may be added to one or more components in the form in which it is supplied or neat. Alternatively, the catalyst may be added to one or more of the components as a solution or dispersion. For example, the catalyst may be diluted in a solvent or polyol component of the reaction prior to use. Suitable solvents may include, but are not limited to, alcohols (such as ethanol), aromatic solvents (such as xylene), or other organic solvents (such as butyl acetate or methoxypropylacetate).

A polymeric drug-device unit of this invention may comprise (or consist essentially of, or consist of) a polyurethane block copolymer made from the starting polymer compositions identified in Table 1 below:

Table 1: An example recipe of starting polymer compositions for making a polyurethane block copolymer for use in a vaginal ring.

| | | |
|---|---|---|
| Polypropylene glycol 2000 | Polymer backbone | 868.6 mg |
| Polyethylene glycol - polypropylene glycol 2000 | Polymer backbone | 306.0 mg |
| Dicyclohexylmethane 4,4'-diisocyanate | Polymer chain linker | 884.8 mg |
| 1,5-Pentanediol | Polymer chain extender | 290.3 mg |
| Bismuth neodecanoate | catalyst | 2.4 mg |

The term "starting polymer compositions" embraces those components and/or compositions used in, for example, reactive extrusion and/or other polymerisation processes to prepare the polyurethane block copolymers of this invention. Moreover, while the term encompasses those starting compositions identified in Table 1, depending on the polymer to be made, other starting compositions and/or amounts may be used.

The polymeric drug-device unit may comprise one or more of the polyurethane block copolymers described herein. The polymeric drug-device unit may comprise a monolithic-type or single matrix-type structure comprising one or more of the described polymers. Alternatively, the polymeric drug-device unit may comprise a reservoir type structure. For example, the polymeric drug-device unit may comprise a layered structure, each layer comprising one or more of the polymers described herein. In such embodiments, an inner layer may be loaded with an active agent, such as quinagolide. A reservoir type polymeric drug-device unit may comprise an inner core structure or layer comprising (or

consisting essentially of, or consisting of) one or more of the polyurethane block copolymers described herein. The polymeric drug-device unit may further comprise an outer layer as a sheath or coating which fully, substantially or at least partially covers or envelopes the inner core structure or layer. The outer layer may comprise, consist essentially or consist of one or more of the polymeric block copolymers described herein. The inner core structure or layer may comprise the active agent (for example quinagolide). The active agent may be absent from the outer layer, sheath or coating. Without wishing to be bound by theory, it is believed that an advantage of such a reservoir type structure is that it can significantly improve the control of the initial and long term release characteristics of the device.

The inner core may comprise, consist essentially of or consist of an extrudable substrate. The extrudable substrate may take the form of a paste or a gel and may comprise a mixture of polymeric materials, pharmaceutical grade polymers, excipients, diluents and the like. These extrudable substrates may be compounded with active agent (for example quinagolide) and fed, inserted or packed into a hollow tube comprising one or more of the polyurethane block copolymers described herein.

Layered structures of the type described above may be formed via a coextrusion process or co-injection moulding process. Alternatively, a reservoir type polymeric drug-device unit may be formed via a combination of a tube extrusion and tube filling process.

The inventors have discovered that the polyurethane block copolymers described herein not only facilitate the sustained and continuous delivery of pharmaceutically active agents such as quinagolide, but they also provide polymeric drug-device units which better control the initial release of the loaded active agent. For example, the polymers and/or polymeric drug-device units of this invention may limit or reduce any "burst release" of the pharmaceutically active agent.

The term "burst release" refers to a rapid and/or somewhat uncontrolled release of a pharmaceutically active agent from a polymeric drug-device unit over a relatively short period of time. The burst release of a pharmaceutically active agent from a polymeric drug-device unit may be particularly prominent in the initial stages of use and/or after placement in a release medium. While any "burst release" effect may be transient and/or short lived, it is a particular problem for controlled release polymeric drug-device units as the initial high (burst) dosage can have an adverse pharmacological effect and may reduce the effective lifetime of a controlled release polymeric drug-device unit.

The present invention is based, in part, on the observation that pharmaceutical polymeric drug-device units which comprise polymers of this invention, exhibit better control over the release of pharmaceutically active agents dispersed or contained therein. For example, polymeric drug-device units which comprise any of the polymers disclosed herein, exhibit, in use (for example after the intravaginal administration to a subject in need thereof), reduced burst release of any pharmaceutical agents dispersed or contained therein. This is particularly true of polymeric drug-device units which contain or have dispersed therein, quinagolide; in such devices the release of the quinagolide is better controlled and less subject to any burst release phenomenon as described above.

The polymeric drug-device units of this invention may comprise polyurethane block copolymers which restrict or contain any burst release of a pharmaceutically active agent to a maximum burst of approximately 90 $\mu$g, approximately 75 $\mu$g or especially approximately 50 $\mu$g within the first day after use (i.e. intravaginal administration) and/or placement in a release medium. For example, the polymeric drug-device units of the invention can have a maximum burst release of about 50 $\mu$g on day one after administration to a patient.

The polymeric drug-device units of this invention may comprise polyurethane block copolymers which restrict or contain an initial burst release of an active agent relative to the steady state release of that agent. A quotient calculated by dividing the percentage release over an initial 24 hour period by the percentage release over a later period (e.g. a period equating to 7- 14 days after administration) may provide an indication of the relative magnitude of the burst release. For example, a lower release quotient may indicate a reduced burst release relative to the steady state release. The polymers described herein may provide a quotient between 0.05 and 10. In some examples, the polymer compositions may provide quotients between about 0.1 and 0.5, or between 0.2 and 0.4. Certain reservoir-type polymeric drug-device units may provide especially low release quotients.

The polyurethane block copolymers described herein may have a molecular weight in the range of between about 45,000 Da and 150,000 Da, or between about 50,000 Da and 100,000 Da. For example, the polyurethane block copolymers may have a molecular weight of about 60,000 Da, about 70,000 Da or 80,000 Da. One of skill would appreciate that the molecular weight of the polyurethane block copolymer may depend on the method of polymer manufacture.

A polydispersity index (PDI) (sometimes referred to as dispersity or heterogeneity index) is a measure of the molecular weight distribution in a polymer sample. The PDI may be calculated using the following formula:

$$PDI = Mw/Mn$$

where Mw is the mass average molecular weight and Mn is the number average molecular weight.

The polyurethane block copolymers may have a PDI in the range of about 1 to 5. In many cases, the polyurethane

block copolymers may have a PDI between about 1 and 2. For example, the polyurethane block copolymers may have a PDI of about 1.5 or 1.6.

As described in more detail below, the polyurethane block copolymers for use in this invention are resilient, deformable/flexible and/or soft. The elastomeric properties of the polymers are due to two primary factors: microphase separation of hard and soft blocks; and the semicrystalline nature of the polymer, whose amorphous phase has a low glass transition temperature. Hard blocks are typically formed from the difunctional compound and diisocyanate. Soft blocks are typically formed from the poly(alkylene oxide) and, optionally, the poly(alkylene oxide) block copolymer moieties. The elasticity may depend on the ratio of hard to soft blocks and may be represented by Shore hardness measurements. Alternatively or additionally, the elasticity of a polymer may be determined by tensile measurements.

In some embodiments, the hard block may comprise between 30 and 70 weight%, particularly between 40 and 60 weight%, of the total weight of the polymer. Conversely, the soft block may comprise between 30 and 70 weight%, particularly between 40 and 60 weight%, of the total weight of the polymer. In preferred embodiments, the polymer may comprise 50 weight% of the hard block and approximately 50 weight% of the soft block, based on the total weight of the polymer.

The polyurethane block copolymers may have a glass transition temperature (Tg) between about -60 and -20 °C. For example, the polyurethane block copolymers may have a glass transition temperature about -40 °C. The crystalline melting temperature (Tm) of the polyurethane block copolymers may increase with the amount of hard block present in the polymer. The crystalline melting temperature of the polyurethane block copolymer may be between about 10 and 50 °C. For example, the crystalline melting temperature may be about 20 °C, about 25 °C or about 30 °C.

The polymeric drug-device units of this invention may comprise polyurethane block copolymers which not only facilitate the desired drug elution profile (i.e. substantially, continuous, sustained delivery without significant burst release), but may exhibit mechanical properties that suit, facilitate or permit use and/or location in a vaginal cavity. For example, the polyurethane block copolymers for use in the polymeric drug-device units of this invention may be resilient, deformable, soft and/or flexible. The polyurethane block copolymer may exhibit a degree of memory such that it can be deformed to enable fitting and insertion and then released to substantially resume its original shape when *in situ.* The polymeric drug-device unitmay adapt and/or conform to the internal profile and/or contours of the vaginal cavity. The soft, flexible, deformable and resilient nature of the polymers for use, ensures that polymeric drug-device units containing the same are not only comfortable to wear but remain in place during and despite user/patient movement.

In order to provide a polymeric drug-device unit suitable for use, location and/or retention in a vaginal cavity, the mechanical properties of a commercially available product (NuvaRing®, Merck) were used as a comparison (as shown in the table 2 below).

Table 2: Mechanical properties of commercially available intravaginal ring

| Product | Elastic Modulus (MPa) | Tensile Stress at 500% Point (MPa) | Max Load | Stress at Max Load | Strain at Max Load (%) |
|---|---|---|---|---|---|
| Nuvaring® | 13.63 | 4.72 | 76.02 | 6.67 | 893.35 |

Without being limited to any particular polyurethane block copolymer, the polymers for use in this invention may have elastic modulus and/or tensile values that are similar to those of NuvaRing®.

The polymeric drug-device unit may have an elastic modulus between about 5 and 100 MPa. For example, the polymeric drug-device units may have an elastic modulus between about 5 and 30 MPa. The polymeric drug-device unit may have an elastic modulus between about 10 and 20 MPa. In some cases, the polymeric drug-device unit may have an elastic modulus between about 10 and 20 MPa when in a hydrated state.

The polyurethane block copolymers for use in this invention may easily be (injection) moulded, extruded or otherwise formed into tubes with a cross-sectional diameter of anywhere between about 1mm and about 10mm. For example, the cross-sectional diameter of the polymer tubes may be about 2mm, 3mm, 4mm, 5mm, 6mm, 7mm, 8mm or 9mm.

The polymeric drug-device units of this invention may take the form of rings which, by virtue of their polymer composition are soft, flexible, resilient and/or deformable in nature. The rings may be made of joined tubular lengths of polymer. The rings may have a regular or variable cross-sectional diameter as described above. Vaginal rings of this invention may be is toroidal in shape. An intravaginal polymeric drug-device unit which is in the form of a ring, may have an outer (major) diameter ranging from about 40 mm to 80 mm (e.g. from about 50 mm to 70 mm, or about 50 mm or 60 mm).

For example, rings for use in the treatment and/or prevention of endometriosis may comprise a quinagolide loaded polyurethane block copolymer as described herein, which quinagolide loaded polyurethane block copolymer takes the form of a soft, flexible ring having a cross-sectional (minor) diameter of about 4mm.

While the polymeric drug-device units of this invention are generally rings for (intra)vaginal use, in other embodiments, polymeric drug-device units of the invention may include suppositories, pessaries for vaginal use, buccal inserts for oral administration, patches for transdermal administration etc.

The polymeric drug-device units of the present invention may comprise quinagolide (or a pharmaceutically acceptable salt thereof: for example quinagolide hydrochloride) at a dose of about 25 to about 15000 micrograms ($\mu$g), or about 200 to 5000 $\mu$g. For example the polymeric drug-device unit may comprise quinagolide at a dose of about 400-1500 $\mu$g. Typically, about 200 $\mu$g, about 400 $\mu$g, about 800 $\mu$g or about 1200 $\mu$g quinagolide is contained (or dispersed) within a polymeric drug-device unit of this invention.

In use, the polymeric drug-device units of this invention may demonstrate or achieve a continuous release of quinagolide to the vaginal tissues. One of skill will appreciate that the magnitude or amount of quinagolide continuously released from a polymeric drug-device unit of this invention will vary depending on the amount loaded into and/or dispersed within the polymeric drug-device unit. Typically, though, a polymeric drug-device unit of this invention may continuously release anywhere between about 1 and about 50 $\mu$g quinagolide/day. The polymeric drug-device unit may continuously release about 5, about 10, about 15, about 20 or about 30 $\mu$g quinagolide/day. The polymeric drug-device unit may continuously release at least about 5, at least about 10, at least about 15, at least about 20 or at least about 30 $\mu$g quinagolide/day. As another example, the drug-device unit may continuously release at most about 45, at most about 40, at most about 35, at most about 30 or at least about 25 $\mu$g quinagolide/day.

Alternatively or additionally, the polymeric drug-device units of the invention may be used to achieve a therapeutically effective plasma concentration of quinagolide in a patient without adverse and/or toxic effects. For example, the polymeric drug-device units may provide a concentration of quinagolide of less than or equal to about 50 pg/ml in the plasma. The polymeric drug-device units may provide a substantially constant level of quinagolide in the blood plasma of between about 1 and 50 pg/ml, e.g. between about 1 and 20 pg/ml over an extended period of time (e.g. over 21 days, over 28 days or over 35 days). The substantially constant plasma concentration of quinagolide may be achieved within 1 to 48 hours after administration (e.g. within approximately 24 hours after administration).

Therefore, without being bound by theory, the polymeric drug-device units of the invention may provide a safer method of administering quinagolide to a patient. The polyurethane block copolymers disclosed herein modulate an initial burst release and a steady state release of quinagolide within 12-36 hours (e.g. within about 24 hours) after initial administration of the polymeric drug-device unit. Further, using the polymeric drug-device units of the invention, a substantially constant level of quinagolide may be achieved in the blood plasma over an extended period of time (e.g. over 21 days, over 28 days or over 35 days).

The quinagolide may be loaded into the polymeric drug-device unit as a granulated formulation, e.g. a wet granulated formulation. Such formulations of quinagolide may act to bind quinagolide and further impede the release of quinagolide from the polymeric drug-device unit in use. Thus, the formulation of quinagolide may assist in controlling (e.g. minimising) both the initial and/or long term release of quinagolide from the polymeric drug-device unit.

One of skill will appreciate that in order to provide a wet granulation formulation of an active agent, a granulation or wetting liquid may be added to the powdered agents. Agitation of the liquid/powder mix results in the provision of wet granules which may then be dried for use. The granulation or wetting liquid may comprise a solvent, for example a volatile solvent. The solvent may comprise water, alcohols (e.g. isopropyl alcohol (IPA)) or mixtures thereof. Once wet, the material to be granulated may be passed through a mesh in order form granules.

Quinagolide for use may be mixed, combined and/or formulated with one or more excipients. The excipients may be selected from natural polymers, cellulose (such as microcrystalline cellulose), and derivatives thereof (such as ethyl cellulose, (hydroxypropyl)methyl cellulose (HPMC) and hydroxypropyl cellulose (HPC)). Other excipients that may be used include polysaccharides (such as pregelatinised starch and pullulan), Zein) and polyvinylpyrrolidone (PVP). For example, quinagolide may be formulated with microcrystalline cellulose (such as Avicil®) and ethyl cellulose.

The granulated formulation may comprise between about 1 and 99% by weight of excipients. For example, the granulated formulation may comprise between about 50 and 99% by weight of excipients. In some cases, the granulated formulation may comprise between about 5-15% by weight of ethyl cellulose (e.g. 7% by weight) and/or about 50-95% by weight of microcrystalline cellulose.

To further assist in loading the quinagolide into the polyurethane block copolymer, an antistatic additive may be used. Such an additive may be of particular use when loading the quinagolide using a hot melt extrusion method. In such processes, the active agent (usually in granular or powder form) is generally dispensed into a polymer feed via a gravimetric feeder. The use of an antistatic additive may improve the flow of the active agent from the gravimetric feeder. Thus, this additive may assist in providing increased uniformity of the active agent (quinagolide) in the polyurethane block copolymer.

The antistatic agent may be fumed silica (e.g. Aerosil). A granulated formulation of quinagolide may comprise between about 0.5 and 5% by weight of the antistatic agent. For example, the antistatic agent may be present in an amount of approximately 1.5% by weight of the granulated formulation.

In view of the above, by way of further example, the polymeric drug-device units of this invention may comprise granulated quinagolide formulations having the following compositions (the granulated quinagolide formulations being

formed by, for example, wet granulation):

Table 3: Exemplary granulated quinagolide formulations

| Component | Amount per vaginal ring | | |
|---|---|---|---|
| Quinagolide hydrochloride | 400 μg* | 800 μg* | 1200 μg* |
| Microcrystalline cellulose | 43.52 mg | 43.12 mg | 42.72 mg |
| Ethyl cellulose | 3.36 mg | 3.36 mg | 3.36 mg |
| Hydrophilic fumed silica | 0.72 mg | 0.72 mg | 0.72 mg |
| *equivalent quantities of quinagolide are 366.2 μg, 732.5 μg and 1098.7 μg for the 400 μg, 800 μg and 1200 μg doses of quinagolide hydrochloride respectively. | | | |

The invention also provides a method of producing the polyurethane block copolymer. The polyurethane block copolymer may be manufactured via a reactive extrusion process or via a batch process.

The method may comprise melting and drying the poly(alkylene oxide), the difunctional compound and optionally the block poly(alkylene oxide) copolymer prior to the reaction. For example, these components may be dried at a temperature of 85°C to 100°C under vacuum. These components are generally dried separately.

The method may comprise mixing, in any suitable order, starting components (a), (b), (c) and (d). For example, components (a), (b) and (d) may be combined together prior to the addition of (c). Alternatively, the method may comprise mixing components (a), (c) and (d) prior to the addition of the difunctional compound (component (b)).

The invention further provides a method for producing the polymeric drug-device unit. The method may comprise loading the quinagolide into a polyurethane block copolymer. The quinagolide may be loaded into the polymer via a hot melt compounding process. For example, the hot melt compounding process may be a hot melt extrusion process. Prior to the loading step, the quinagolide may be formulated into granules as is disclosed herein.

As stated, the drug-device units of this invention may take the form of rings for intravaginal use. The rings may be formed by an extrusion process in which short lengths of extruded polymer are formed into rings with the ends being joined by any suitable method including, for example gluing, welding, laser welding and fastening. Alternatively, ring type drug-device units may be formed by a process comprising injection moulding.

In addition to the above, a second aspect of this invention provides a polymeric drug-device unit of this invention for use in the treatment and/or prevention of endometriosis.

In a third aspect, the invention may further provide the use of a polymeric drug-device unit of this invention in the manufacture of a medicament for the treatment and/or prevention of endometriosis.

Moreover, in a fourth aspect, the invention provides a method of treating and/or preventing endometriosis, said method comprising administering to a subject in need thereof, a polymeric drug-device unit of this invention. The method may comprise insertion of the drug-device unit into the vagina of a subject (for example a patient), leaving the device *in situ* for a predetermined or prescribed period of time and thereafter removing said unit. The subject may be administered a further drug-device unit.

A subject in need thereof or indeed a subject to be administered a polymeric drug-device unit, composition or medicament of this invention may be any subject suffering from or exhibiting the symptoms of endometriosis. Those susceptible or predisposed to developing endometriosis may also be administered a device or composition/medicament of this invention.

In a fifth aspect, the invention further provides a kit comprising a polymeric drug-device unit as described herein and an applicator.

The applicator may facilitate insertion of the polymeric drug-device unit into a patient. For example, the applicator may facilitate insertion of the polymeric drug-device unit (such as a vagina ring) into a vaginal cavity.

In the kit, the polymeric drug-device unit may be pre-loaded into or onto the applicator.

The kit may be contained in sterile packaging.

It will be appreciated that those features described in detail for the first aspect of the invention may be equally applicable to the second, third, fourth and fifth aspects of the invention.

**Detailed Description**

The present invention will now be described in detail with reference to the following Figures which show:

**Figure 1:** General overview of an example manufacturing process for a polymeric drug-device unit according to one

embodiment of the invention.

**Figure 2:** *In vitro* Dissolution profiles showing release of quinagolide from various drug loaded polyurethane block copolymers (1.0% w/w, 4x4mm Blocks) over a 28 day period.

**Figure 3:** *In vitro* Dissolution profiles showing release of quinagolide from drug loaded polymers RLST0183 and RLST0157.

**Figure 4:** *In vitro* Dissolution profiles showing release of quinagolide from drug loaded polymers RLST0072 and RLST0154 (0.5% w/w, 4x4mm Blocks) over a 28 day period.

**Figure 5:** *In vitro* Dissolution profiles showing release of quinagolide from further drug loaded polyurethane block copolymers compared to RLST0072 and RLST0154 over a 10 day period.

**Figure 6:** *In vitro* Dissolution profiles for batches QH12019, QH12020 and QH12022 showing release of quinagolide over a 20 day period.

**Figure 7:** Release of quinagolide *in vivo* from batches QH12020 and QH12022 over a 28 day period in a first study in sheep.

**Figure 8:** Release of quinagolide *in vivo* from batches QH13005 and QH13006 over a 28 day period in a second study in sheep.

**Figure 9:** Average daily rate of quinagolide hydrochloride release *in vivo* from batches QH12020, QH12022, QH13005 and QH13006, as found in the first and second sheep studies over a 28 day period.

**Figure 10:** Plasma concentrations of quinagolide (Q) and active metabolites (M1 and M2) over a 28 day period during the first and second sheep studies.

**Figure 11:** Dissolution profiles of co-extruded batches QH13017-QH13024 showing release of quinagolide over a 28 day period

**Figure 12:** *In vivo* Release profile of vaginal rings in sheep. Plasma concentrations of quinagolide (Q: 400ug (panel A): 800ug (panel B): 1100 ug (panel C)) and active metabolites (M1 and M2) over a 35 day period.

**Figure 13:** Quinagolide metabolites M1 and M2.

**Manufacturing Process Overview**

A general overview of an example manufacturing process for a polymeric drug-device unit according to this invention is shown in Figure 1.

The five principal stages of the manufacturing process are shown in boxes 100, 105, 110, 115 and 120.

The first stage involves preparation of raw materials and catalyst (box 100).

The polyurethane block copolymer may be manufactured using reactive extrusion, batch processing or any other suitable method (box 105).

Separately, and optionally in parallel, the active agent may be prepared as a granular formulation (box 110).

The next stage comprises loading the polymer with the active agent (box 115). The granular drug is uniformly incorporated or compounded with the polymer.

The fifth stage of the process comprises formation of the ring product. The rings may be formed by any number of suitable methods including, for example, bonding together the ends of extruded cylindrical polymer tubes using a medical-grade adhesive or welding, for example heat welding or laser welding. Alternatively, the ring may be formed via an injection moulding process.

The ring product is then packaged to allow storage. For example, the ring product may be placed in packaging that protects against moisture and/or gas ingress.

Each of the stages of the manufacturing process will be further described in the following examples.

**Polymer synthesis**

*Preparation of raw materials for polymer manufacture*

The starting polymer compositions (the poly(alkylene oxide), the difunctional compound and (where present) the poly(alkylene oxide) block copolymer) were dried to remove water by heating under vacuum.

The difunctional isocyanate was stirred and heated under nitrogen prior to use.

*Preparation of the catalyst*

The catalyst may be prepared for use as a dispersion or solution or used neat. Any of the catalysts described herein may be used.

For example a bismuth catalyst (BiCat) (e.g. bismuth neodecanoate) (10 g) was dissolved in ethanol. 1,5-pentanediol (100 g) was added to the solution and then the ethanol removed using a rotary evaporator to provide a

dispersion of BiCat in 1,5-pentanediol (10 wt%).

*Manufacture of polymer by a reactive extrusion process*

The reactants (the poly(alkylene oxide), the difunctional compound, the difunctional isocyanate and (where present) the poly(alkylene oxide) block copolymer) were dispensed into an extruder using a liquid feed system. The catalyst or the catalyst dispersion was simultaneously dispensed into the extruder from volume calibrated syringes using a syringe pump.

Using methods that would be known to persons skilled in the art, the rate of flow of each of the individual liquid streams into the extruder was fixed to ensure the final polymer contained the appropriate proportion of each of the starting composition materials.

The polyurethane block copolymer was discharged from the extruder as a strand. The strand was conveyed through a water bath and cooling coils into a pelletiser. After pelletisation, the polymer pellets were stored at room temperature until required.

*Manufacture of polymer by a batch process*

A typical batch reactor comprises a vessel and an agitator which may be jacketed with a heating/cooling system. Once an initial temperature had been reached, the reactor was charged with the reactants and catalyst. Alternatively or additionally, the temperature was adjusted after the reactants had been fed into the reactor vessel. The reaction temperature and torque were monitored throughout the duration of the polymerisation. The polymerisation was considered complete when the torque level reached equilibrium. The polymer was then discharged from the reactor and pelletised.

*Preparation of granular drug formulation*

Quinagolide hydrochloride may be prepared as a granular drug formulation using, for example, a wet granulation process, as described below.

Quinagolide hydrochloride (QH) was blended directly with microcrystalline cellulose (e.g. Avicel PH101). In those cases where lower doses of quinagolide hydrochloride were required, the quinagolide hydrochloride was added as a solution in isopropanol (IPA) to the microcrystalline cellulose. A mixture of ethyl cellulose in IPA was then added to the quinagolide hydrochloride/microcrystalline cellulose blend.

The wet mixture was passed through a granulator sieve to form granules. The granules were dried in an oven.

Once dried, the granules were mixed with hydrophilic fumed silica (e.g. Aerosil 200VV) before being further reduced in size using a finer granulator sieve.

The final material was then hand sieved.

Each batch of granules was tested to ensure content uniformity and to monitor the levels of residual water and IPA.

Calculation of Granule Composition

As will be appreciated, the exact quantities of the quinagolide salt and other components used during the preparation of the granules will be dependent on the desired dose in the final drug-device unit. To obtain a desired dose of active agent in the final drug-device unit, the skilled person would need to account for the target throughput rate of the extrusion process in the subsequent drug loading step, the concentration of active agent in the granule and also the target drug-device unitweight.

By way of example only, the following parameters have been adopted:

- Target vaginal ring weight: 2.4 g
- Target concentration of Quinagolide HCl granule in polymer: 2%
- Target throughput rate of drug feeder during extrusion: 40 g/hour
- Target throughput rate of polymer feeder during extrusion: 1960 g/hour
- Batch size of Quinagolide HCl granule being prepared: 300 g
- Target doses of quinagolide HCl in the vaginal ring: 400 mcg, 800 mcg and 1200 mcg

The quinagolide hydrochloride concentration required for this particular batch size, ring weight and target doses may be calculated as shown in Table 4 below:

Table 4: Calculation of quinagolide hydrochloride concentration for target doses of 400 mcg, 800 mcg and 1200 mcg

| QH Dose in Ring (mcg) | Ring Weight (g) | %w/w QH in Ring | Granule Concentration in Polymer (%) | %w/w QH in Granule | Batch Size (g) | Quantity of QH in Granule Batch (g) |
|---|---|---|---|---|---|---|
| A | B | C=A/(B x 10000) | D | E = (C x 100) / D | F | G= (F x E)/100 |
| 400 | 2.4 | 0.01667 | 2 | 0.8334 | 300 | 2.500 |
| 800 | 2.4 | 0.03333 | 2 | 1.6665 | 300 | 5.000 |
| 1200 | 2.4 | 0.05000 | 2 | 2.5000 | 300 | 7.500 |

The concentrations and quantities of the other excipients present in the example granule batches are shown in Tables 5, 6 and 7.

Table 5: Target dose of 400mcg of quinagolide hydrochloride in vaginal ring

| Material | %w/w in Granule | Quantity Required (g) |
|---|---|---|
| Quinagolide HCl | 0.8334 | 2.500 |
| Avicel PH101 | 90.667 | 272.00 |
| Ethyl Cellulose | 7.000 | 21.00 |
| Aerosil 200VV | 1.500 | 4.500 |
| Total Solids | 100.00 | 300.00 |
| Isopropyl Alcohol | 53% of solids content | 159.00 |

Table 6: Target dose of 800mcg of quinagolide hydrochloride in vaginal ring

| Material | %w/w in Granule | Quantity Required (g) |
|---|---|---|
| Quinagolide HCl | 1.6665 | 5.000 |
| Avicel PH102 | 89.833 | 269.50 |
| Ethyl Cellulose | 7.000 | 21.00 |
| Aerosil 200VV | 1.500 | 4.500 |
| Total Solids | 100.00 | 300.00 |
| Isopropyl Alcohol | 53% of solids content | 159.00 |

Table 7: Target dose of 1200mcg of quinagolide hydrochloride in vaginal ring

| Material | %w/w in Granule | Quantity Required (g) |
|---|---|---|
| Quinagolide HCl | 2.500 | 7.500 |
| Avicel PH102 | 89.000 | 267.00 |
| Ethyl Cellulose | 7.000 | 21.00 |
| Aerosil 200VV | 1.500 | 4.500 |
| Total Solids | 100.00 | 300.00 |
| Isopropyl Alcohol | 53% of solids content | 159.00 |

*<u>Loading of active agent into the polymer using hot melt extrusion</u>*

The granules comprising quinagolide hydrochloride were compounded with the pre-prepared polymer pellets using a hot melt extrusion process. Hot melt extrusion is a widely used method of loading active agents into polymers in the pharmaceutical industry.

The granular drug formulation and the polymer pellets were charged into gravimetric feeders and dispensed into the extruder at a rate to provide the desired dose of active agent in the final ring product. An appropriate set of compounding screws, screw speed and temperature profile were also selected. As will be appreciated, the exact parameters selected may be dependent upon the nature of the polymer compositions, granules and target dose in the final product. The appropriate selection of such parameters would be well within the capabilities of the skilled person.

After extrusion, the drug loaded polymer strand was passed through a cutting unit and cut to the required length. The length of the strand determines the circumference of the final ring product. Therefore the required length will be dependent upon the target dimensions of the final ring product.

The cut strand lengths were then sealed in foil bags and stored in a freezer until the subsequent ring formation process.

<u>Ring formation</u>

A primer was dispensed onto the cylindrical ends of the polymer strand from a pressurised spray dispenser, before application of a medical grade adhesive to a first end of the strand using a peristaltic pump dispenser. The first end of the strand was then joined to the second end of the strand to form the vaginal ring product.

As will be appreciated, other methods of joining the ends of the strand may be used to form the vaginal ring product. For example, the ends may be welded together by a heat or laser welding process. Alternatively the ring may be formed via injection moulding. In such cases, the extruded polymer strand can be pelletised, before being transferred to an injection moulder. In such cases, the polymer is formed directly into a ring shape.

After formation, the ring products were packaged in an individual foil bag.

**Polymer Compositions**

The polyurethane block copolymers are obtainable by reacting together components:

(a) a poly(alkylene oxide);
(b) a difunctional compound;
(c) a difunctional isocyanate; and
(d) optionally a block copolymer comprising poly(alkylene oxide) blocks.

The starting polymer compositions identified in Table 8 have been used to prepare polyurethane block copolymers, which were subsequently investigated for use in drug-device units comprising quinagolide.

The relative amounts and the nature of these components are indicated in Table 8.

Table 8: Example starting polymer compositions used to prepare polyurethane block copolymers for use as drug-device units comprising quinagolide.

| Polymer batch | Starting polymer Composition (wt %) | Stoichiometry (a) : (b) : (c) : (d) |
|---|---|---|
| RLST0027 | PPG2000 26.9%; decanediol 15.6%; DMDI 30.6%; PPG-PEG-PPG2000 26.9%. | 0.15 : 1 : 1.3 : 0.15 |
| RLST0047 | PPG2000 24.5%; decanediol 17.8%; DMDI 33.2%; PPG-PEG-PPG2000 24.5%. | 0.12: 1 : 1.24 : 0.12 |
| RLST0072 | PPG2000 22.5%; decanediol 19.6%; DMDI 35.4%; PPG-PEG-PPG2000 22.5%. | 0.1 : 1 : 1.2 : 0.1 |
| RLST0098 | PPG2000 27.3%; pentanediol 10.9%; DMDI 34.5%; PPG-PEG-PPG2000 27.3%. | 0.135 : 1 : 1.3 : 0.135 |
| RLST0044 | PEG2000 10.8%; decanediol 15.6%; DMDI 30.6%; PEG-PPG-PEG2000 43.0%. | 0.06 : 1 : 13 : 0.24 |
| RLST1015 | PPG2000 35.0%; decanediol 12.2%; | 0.25 : 1 : 1.5 : 0.25 |

(continued)

| Polymer batch | Starting polymer Composition (wt %) | Stoichiometry (a) : (b) : (c) : (d) |
|---|---|---|
| | HMDI 17.7%; PPG-PEG-PPG2000 35.0%. | |
| RLST0154 | PPG2000 33.1%; pentanediol 11.1%; DMDI 35.1%; PPG-PEG-PPG2000 20.7%. | 0.155 : 1 : 1.252 : 0.097 |
| RLST0155 | PPG2000 28.0%; pentanediol 13.9%; DMDI 41.0%; PPG-PEG-PPG2000 17.1%. | 0.1048 : 1 : 1.169 : 0.0643 |
| RLST0156 | PPG2000 34.7%; pentanediol 11.7%; DMDI 36.3%; PPG-PEG-PPG2000 17.4%. | 0.1549 : 1 : 1.232 : 0.0774 |
| RLST0157 | PPG2000 30.7%; pentanediol 13.9%; DMDI 41.0%; PPG-PEG-PPG2000 14.4%. | 0.1151: 1 : 1.169: 0.0539 |
| RLST1040 | PPG2000 51.9%; decanediol 12.2%; HMDI 17.7%; PPG-PEG-PPG2000 18.2%. | 0.37:1 : 1.5 : 0.13 |
| RLST1041 | PPG2000 55.9%; decanediol 9.4%; HMDI 15.4%; PPG-PEG-PPG2000 19.4%. | 0.52 : 1 : 1.7 : 0.18 |
| RLST0183 | PPG2000 45.1%; pentanediol 13.9%; DMDI 41.0% | 0.169 : 1 : 1.169 |
| RLST0208 | PPG2000 41.5%; pentanediol 10.9%; DMDI 34.5%; PPG-PEG-PPG2000 13.1%. | 0.199: 1 : 1.262: 0.0631 |
| RLST0207 | PPG2000 43.7%; pentanediol 10.2%; DMDI 33.1%; PPG-PEG-PPG2000 13.0%. | 0.2237 : 1 : 1.290 : 0.0667 |
| RLST0209 | PPG2000 47.0%; pentanediol 9.2%; DMDI 31.0%; PPG-PEG-PPG2000 12.9%. | 0.2667 : 1 : 1.340 : 0.0730 |
| RLST0210 | PPG2000 37.0%; pentanediol 12.3%; DMDI 37.7%; PPG-PEG-PPG2000 13.0%. | 0.156 : 1 : 1.211 : 0.056 |
| RLST0211 | PPG2000 35.0%; pentanediol 13.0%; DMDI 39.0%; PPG-PEG-PPG2000 13.0%. | 0.1404 : 1 : 1.193 : 0.0523 |
| RLST0212 | PPG2000 36.0%; pentanediol 12.7%; DMDI 38.3%; PPG-PEG-PPG2000 13.0%. | 0.148 : 1 : 1.201 : 0.054 |
| RLST0213 | PPG2000 38.0%; pentanediol 12.0%; DMDI 37.0%; PPG-PEG-PPG2000 13.0%. | 0.1646 : 1 : 1.221 : 0.0564 |

The block co-polymers used in the example compositions were as follows:

PPG-PEG-PPG2000 comprises approximately 50% by weight of PEG. For example, a block co-polymer having a percentage weight ratio of approximately 25:50:25 of its constituent blocks.
PEG-PPG-PEG2000 comprised approximately 10% by weight of PEG. For example, a block co-polymer having a percentage weight ratio of approximately 5:90:5 of its constituent blocks.

**Evaluation of polyurethane block copolymers**

Dissolution testing

A dosage form when placed into a vessel containing liquid media will release drug in a defined manner dictated by the formulation. This process, known as dissolution, can be used as an *in vitro* marker of the mechanism of release in the body. Sampling is carried out at regular intervals and the amount of drug in the samples is analysed by spectrophotometer or HPLC. The data are normally represented as the release of labelled content against time.

Tensile Testing

Films for each polymer were prepared using a 2 mm mould on a custom made hot-press. The temperature set

on the hot-press varied depending on the polymer composition to ensure a linear melt and a suitable film was obtained. The 2 mm polymer films were removed from their moulds and punched with a Ray-Ran hand operated cutting press to make a dog-bone shape of type 2 dimension as outlined in the ISO standard (International Organisation Standardisation) 37:2005(E) or a cylindrical length sample.

An Instron 3343 mechanical tester was used and the samples were tested to destruction at a rate of 200 mm/min and the stress-strain curves recorded. The capacity of the load cell used for this test was 1000 N.

Tensile testing was also carried out on formed rings in the dry, hydrated, blank and drug loaded state.

Dynamic Mechanical Analysis (DMA)

A dynamic mechanical analyser was used to record storage and loss modulus (G' and G", respectively) and loss tangent (G'/G") as a function of temperature. The samples were cooled below the glass transition temperature before being heated at a rate of 2 °C/min. Samples (1 mm) were prepared in accordance with the method outlined above under "Tensile Testing").

Gel Permeation Chromatography (GPC)

Molecular weight analysis (Mw, Mn and polydispersity index (PDI)) of the polymers was carried out by Gel Permeation Chromatography (GPC.) Each sample was dissolved in tetrahydrofuran (THF.) The system eluent was converted to THF at least 24 hours prior to samples being run. The equipment was calibrated using the polystyrene narrow and broad standards and set up with a 2 x PLgel MIXED-C, 5 μm, 300 x 7.5 mm column (including a guard column) before use. The samples were run at a flow rate of 1 ml $min^{-1}$.

Release of Quinagolide

To provide an initial analysis of the suitability of the polyurethane block copolymers for the delivery of quinagolide, various polymers were loaded with quinagolide and their release profiles assessed.

Exemplary drug loaded polyurethane block copolymers were prepared by compounding quinagolide and pellitised polyurethane block copolymer in a batch compounder. The resultant 1.0% w/w drug loaded polymers were processed into sample blocks (4x4 mm) and dissolution testing was carried out.

The results are shown in Table 9 and Figure 2.

Table 9: Release of quinagolide from various drug loaded polymer compositions (1.0% w/w, 4x4mm Blocks) over a 28 day period.

| | Polymer batch | drug released in first 24h (%) | drug released between 7 and 14 day (%) | Quotient of 24 h release/ 7-14 day release |
|---|---|---|---|---|
| QH12001 | RLST0027 | 28.2 | 11.6 | 2.4 |
| QH12002 | RLST0047 | 20.4 | 15.2 | 1.3 |
| QH12003 | RLST0072 | 8.6 | 9.9 | 0.9 |
| QH12004 | RLST1015 | 50.0 | 5.7 | 8.8 |
| QH12005 | RLST0098 | 41.8 | 8.9 | 4.7 |
| QH12006 | RLST0044 | 13.6 | 20.7 | 0.7 |

The quotient (of 24 h release/ 7-14 day release) provides a measurement of the "burst release" of an active agent relative to a steady state release. In Table 9, the quotient has been calculated by division of the percentage of drug released in the initial 24 hour period by the percentage of drug released between 7 and 14 days (representing the steady state for a 1 month product)

Polymers RLST0072 and RLST0044 gave lower quotient values indicating that such polymers would be suitable for a release profile with minimal burst release. The other polymers, RLST0027, RLST0047, RLST1015 and RLST0098, gave higher quotient values and so would be useful when a higher initial rate of quinagolide delivery is required.

The release of quinagolide from polymers RLST0183 and RLST0157 is also shown in Figure 3.

As a consequence, polymer batch RLST0154 was developed and its release profile was compared with that of RLST0072. Both polymers were compounded with quinagolide in a batch compounder to produce 0.5% w/w drug

loaded polymers and processed into blocks and dissolution tested (as shown in Table 10 and Figure 4).

Table 10: Release of quinagolide from two drug loaded polymers (0.5% w/w, 4x4 mm Blocks) over a 28 day period.

| | Polymer batch | drug released in first 24h (%) | drug released between 7 and 14 day (%) | Quotient 24 h release/ 7-14 day release |
|---|---|---|---|---|
| QH12012 | RLST0072 | 18.6 | 5.4 | 3.5 |
| QH12013 | RLST0154 | 22.5 | 10.8 | 2.1 |

The results demonstrated that polymer RLST0154 provides a slightly reduced comparative burst release (lower quotient value) and similar release profile when compared to polymer RLST0072.

The dosage of the active agent in the polymer also has an effect on the relative burst release compared to the steady state release of the agent from the polymer. This is exemplified in the different quotient values observed for polymer RLST0072 when loaded with 1.0% w/w and 0.5% w/w of quinagolide (0.9 and 3.5 respectively).

To develop a polymer that provided a slower release rate than RLST0154 and RLST0072, a number of polymers were manufactured by modulating the starting polymer compositions used to prepare polymer RLST0154. The relative performance of these new polymer batches against RLST0072 and RLST0154 was assessed and the results are presented in Table 11 and Figure 5.

Table 11: Release of quinagolide from further drug loaded polymers compared to RLST0072 and RLST0154, including dosage form and loading details.

| | Polymer batch | Dosage form & Loading details | drug released in first 24h (%) | drug released after 7 days (%) | Quotient 24 h release/ 7day release[3] |
|---|---|---|---|---|---|
| QH12013 | RLST0154 | Melt mix blocks 0.05% w/w 48μg/unit | 22.5 | 48.2 | 0.47 |
| QH12024 | RLST0156 | Melt mix blocks 0.05% w/w 51μg/unit | 13.3 | 32.6 | 0.41 |
| QH12025 | RLST0155[1] | Melt mix blocks 0.05% w/w 60μg/unit | 12.9 | 29.5 | 0.44 |
| QH13003 | RLST0157[2] | Extruded rods 0.05% w/w 1824μg/unit | 5.8 | 15.6 | 0.37 |
| [1,2] Manufactured by a reactive extrusion process. [3]Dissolution was stopped after 7 days for QU12023, 024 & 025. Therefore the final column shows the quotient of the 24 hour release over the 7 day release. | | | | | |

**Sheep study trial**

Polymer batches were loaded with quinagolide and manufactured into rings for a sheep study.

Table 12 provides details of the polyurethane block copolymers manufactured and Table 13 provides details of the mechanical properties. It should be noted that hot melt extrusion was used to compound the drug with the polymer and therefore quinagolide was dry blended with Avicel to enable the powder feeder dispensing the drug into the extruder to meet the low doses being targeted with good content uniformity. The hot melt extruded material was manufactured into rings using the process of heat sealing.

Table 12: Polymer batches used for the first sheep study.

| | Polymer batch | Ring dimensions & Loading details | drug released in first 24h (%) | drug released between 7 and 14 day (%) | Quotient 24 h release/ 7-14 day release |
|---|---|---|---|---|---|
| QH12019 | RLST0044 | 5mm Ring Units 0.05% w/w 1840μg/unit | 10.1 | 10.3 | 1.0 |

(continued)

| | Polymer batch | Ring dimensions & Loading details | drug released in first 24h (%) | drug released between 7 and 14 day (%) | Quotient 24 h release/ 7-14 day release |
|---|---|---|---|---|---|
| QH12020 | RLST0072 | 4mm Ring Units 0.05% w/w 2223μg/unit | 6.9 | 7.3 | 0.9 |
| QH12022 | RLST0072 | 5mm Ring Units 0.1% w/w 3430μg/unit | 6.7 | 7.6 | 0.9 |

Table 13: Dry blend formulation details and mechanical properties of formulations used in the first sheep study.

| Batch number | Formulation Detail (Dry Blend) | Mechanical Properties | | | |
|---|---|---|---|---|---|
| | | Elastic Modulus (MPa) | Load at Break (N) | Tensile Stress at Max Load (MPa) | Tensile Stress at 500% (%) |
| RLST0072 | N/A | 10.91 | 334.35 | 17.90 | 786.17 |
| QH12020 | Quinagolide HCl 3.5% w/w Avicel PH101 96.5% w/w | 10.23 | 272.64 | 13.67 | 707.45 |
| QH12022 | Quinagolide HCl 3.5% w/w Avicel PH101 96.5% w/w | 12.72 | 293.84 | 13.86 | 760.43 |

Dissolution profiles for QH12019, QH12020 and QH12022 are shown in Figure 6.

The intravaginal rings were placed in sheep and the amount of quinagolide released *in vivo* was monitored over a 28 day period. The results of this first sheep study are shown in Table 14 below and illustrated in Figure 7.

Table 14: Release of quinagolide *in vivo* over a 28 day period in the first sheep study.

| Batch Number | Dose (mcg) | Quinagolide released (mcg) | Average Daily Release over 28 days (mcg) |
|---|---|---|---|
| QH12020 | 2000 | 1065.5 | 38.1 |
| QH12022 | 3100 | 1461.6 | 52.2 |

For the purposes of comparison, the *in vitro* release of quinagolide from QH12020 and QH12022 is also shown on Figure 7.

A second study in sheep was conducted using polymer batches based on RLST0157. This polymer had been shown to have a slower release profile than RLST0072. Tables 15 and 16 below show the formulation details and mechanical data for the polymers tested.

Table 15: Polymer batches used for second sheep study.

| | Polymer batch | Ring dimensions & Loading details | drug released in first 24h (%) | drug released between 7 and 14 day (%) | Quotient 24 h release/ 7-14 day release |
|---|---|---|---|---|---|
| QH13005 | RLST0157 | 6mm Ring Units 0.03% w/w 1500μg/unit | 5.8 | 5.2 | 1.1 |

(continued)

| | Polymer batch | Ring dimensions & Loading details | drug released in first 24h (%) | drug released between 7 and 14 day (%) | Quotient 24 h release/ 7-14 day release |
|---|---|---|---|---|---|
| QH13006 | RLST0157 | 5mm Ring Units 0.03% w/w 1200μg/unit | 6.4 | 5.9 | 1.1 |

Table 16: Dry blend formulation details and mechanical properties of formulations used in the second sheep study.

| Batch number | Formulation Detail (Dry Blend) | Mechanical Properties | | | |
|---|---|---|---|---|---|
| | | Elastic Modulus (MPa) | Load at Break (N) | Tensile Stress at Max Load (MPa) | Tensile stress at 500% (%) |
| RLST0157 | N/A | 32.32 | 337.41 | 16.51 | 1115.92 |
| | | | | | |
| QH13005 | Quinagolide HCl 2.4% w/w Avicel PH101 97.6% w/w | 32.94 | 411.44 | 14.69 | 1143.55 |
| QH13006 | Quinagolide HCl 2.4% w/w Avicel PH101 97.6% w/w | 34.37 | 306.08 | 15.10 | 1065.77 |

The intravaginal rings were placed in sheep and the amount of quinagolide released *in vivo* was monitored over a 28 day period. The results of this sheep study are shown in Table 17 below and illustrated in Figure 8.

Table 17: Release of quinagolide *in vivo* over a 28 day period in the second sheep study.

| Batch Number | Dose (mcg) | Quinagolide released (mcg) | Average Daily Release over 28 days (mcg) |
|---|---|---|---|
| QH13005 | 1500 | 580.8 | 20.7 |
| QH13006 | 1100 | 464.9 | 16.6 |

For the purposes of comparison, the *in vitro* release of quinagolide from QH13005 and QH13006 is also shown on Figure 8.

The average daily rate of quinagolide hydrochloride release from batches QH12020, QH12022, QH13005 and QH13006, as found in the first and second sheep studies, is further illustrated in Figure 9.

During the first and second sheep trials, the plasma concentration of quinagolide (Q) was monitored over the 28 day period. The plasma concentrations of active metabolites (M1 and M2: see Figure 13) were also monitored in the sheep. The results are illustrated in Figure 10.

It was found that the use of intravaginal rings made from batches QH12022, QH12020 and QH13006 provided substantially constant levels of quinagolide in the plasma over the 28 day period. Further, the quinagolide concentration in the plasma did not exceed 50 pg/ml at any point during the study. The levels of the active metabolites M1 and M2 were present in the plasma at approximately 10-fold lower molar concentrations than the quinagolide.

A further study was carried out in sheep to determine the *in vivo* release over the period of 35 days for polymer rings with a quinagolide load targeted at delivering 5, 10 and 15 μg/day. Table 18 below shows the actual release rates achieved were almost identical to the target and that the initial release on day one has been reduced.

Table 18: *In vivo* Release profile of vaginal rings in sheep

| Dose (μg) | Target release rate (μg/day) | Day 1 release (μg) | Average release from Day 2 to Day 28 (μg/day) | Average release from Day 2 to Day 35 ((μg/day) |
|---|---|---|---|---|
| 400 (QH13067) | 5 | 15 | 4.1 | 5.4 |

(continued)

| Dose (μg) | Target release rate (μg/day) | Day 1 release (μg) | Average release from Day 2 to Day 28 (μg/day) | Average release from Day 2 to Day 35 ((μg/day) |
|---|---|---|---|---|
| 800 (QH13068) | 10 | 29 | 10.9 | 10.4 |
| 1100 (QH13069) | 15 | 35 | 14.8 | 13.9 |

**Reservoir Type drug-device units**

Reservoir type quinagolide vaginal rings were manufactured using an excipient blend of quinagolide HCl with Avicel at a drug concentration of 3.5 % compounded with RLST072 as a core and co-extruded with RLST072 or RLST0047 or RLST0046 as a sheath or cap (which did not contain quinagolide HCl) surrounding the core to form coextruded tubes that were cut to length and formed into rings.

The dissolution data for the reservoir-type rings is shown in Figure 11 and Table 19 below.

Table 19: Composition and release details for the reservoir-type rings.

| | Polymer batch Composition | Ring dimensions & Loading details | drug released in first 24h (%) | drug released between 7 and 14 day (%) | Quotient 24 h release/ 7-14 day release |
|---|---|---|---|---|---|
| QH13017 | RLST0072 core/RLST0072 cap | 3.5mm ring 0.1 %w/w/ 2817μg/unit | 1.9 | 11.4 | 0.35 |
| QH13018 | RLST0072 core/RLST0047 cap | 3.5mm ring 0.1 %w/w/ 2616μg/unit | 2.5 | 8.6 | 0.20 |
| QH13019 | RLST0072 core/RLST0046 cap | 3.5mm ring 0.1%w/w/ 2641μg/unit | 2.6 | 7.5 | 0.22 |
| QH13020 | RLST0072 core/RLST0046 cap | 3.5mm ring 0.1 %w/w/ 2269μg/unit | 1.2 | 6.9 | 0.16 |
| QH13021 | RLST0072 core/RLST0046 cap | 3.5mm ring 0.1 %w/w/ 3822μg/unit | 0.2 | 3.8 | 0.09 |
| QH13022 | RLST0072 core/RLST0047 cap | 3.5mm ring 0.1 %w/w/ 3664μg/unit | 0.7 | 6.1 | 0.13 |
| QH13023 | RLST0072 core/RLST0072 cap | 3.5mm ring 0.1 %w/w/ 3115μg/unit | 1.2 | 7.3 | 0.14 |
| QH13024 | RLST0072 core/ No cap Control | 3.5mm ring 0.1 %w/w/ 4546μg/unit | 5.6 | 8.2 | 0.31 |

It was observed that these reservoir type vaginal rings were able to provide substantially zero order release with little or no burst release and low steady state release. The quotient of the %24 hour release divided by the % drug released between 7 and 14 days for the reservoir-type rings were all extremely low (0.09 to 0.35).

**Modulation of mechanical properties**

Further development work centred round modulation of the mechanical properties of the polymer. It had been found that RLST0157 (having a Young's modulus of approximately 52 MPa) provided a relatively stiff ring polymeric drug-device. There was interest in developing further compositions with decreased stiffness which could prove more comfortable to an end user.

Table 20 below provides the details of the further polymers that were manufactured.

Table 20: Polymers manufactured during the investigation of mechanical properties

| Polymer batch | Starting polymer composition (wt %) | Hard Segment Content (wt%)[1] |
|---|---|---|
| RLST0157 | DMDI 41.0% - pentanediol 13.9% - PPG2000 30.7% - PPG-PEG-PPG2000 14.4%. | 55 |
| RLST0208 | DMDI 34.5% - pentanediol 10.9% - PPG2000 41.5% - PPG-PEG-PPG2000 13.1%. | 45 |
| RLST 0210 | DMDI 37.7% - pentanediol 12.3% - PPG2000 37.0% - PPG-PEG-PPG2000 13.0%. | 50 |
| RLST0211 | DMDI 39.0% - pentanediol 13.0% - PPG2000 35.0% - PPG-PEG-PPG2000 13.0%. | 52 |
| RLST0212 | DMDI 38.3% - pentanediol 12.7% - PPG2000 36.0% - PPG-PEG-PPG2000 13.0%. | 51 |
| RLST0213 | DMDI 37.0% - pentanediol 12.0% - PPG2000 38.0% - PPG-PEG-PPG2000 13.0%. | 49 |
| [1] Hard Segment Content is the combined % by weight of the diol and diisocyanate components. | | |

The mechanical properties of these polymers were tested and the results compared to RLST0157 as shown in Table 21 below.

Table 21: Mechanical properties of various polymers.

| **Elastomer** | **Hard segment (%)** | **Elastic Modulus (MPa)** | **Tensile stress at 500% strain (MPa)** | **Tensile Strain at max (%)** |
|---|---|---|---|---|
| RLST0157 | 57 | 52 | N/A | 844 |
| RLST0211 | 52 | 25.6 | 9.3 | 1267 |
| RLST0213 | 51 | 10.8 | 5.7 | 1523 |
| RLST0210 | 50 | 13.6 | 6.0 | 1831 |
| RLST0208 | 46 | 7.8 | 4.7 | 1880 |

As can be seen from the table above, all the tested polymers exhibited lower elastic modulus values than RLST0157. Based on the mechanical data, RLST0210 was selected for further investigation as a lead polymer for clinical trial manufacture.

**Dynamic Mechanical Analysis**

Dynamic thermal mechanical analysis of samples was performed in tension mode (Table 22).

Table 22. Thermal transitions of example polymers as determined by DMA

| **Polymer batch** | **Hard Segment Content (%)** | **Tg (°C)** | **Tm1 (°C)** |
|---|---|---|---|
| RLST0208 | 46 | -41 | 21 |
| RLST0213 | 49 | -43 | 28 |

(continued)

| Polymer batch | Hard Segment Content (%) | Tg (°C) | Tm1 (°C) |
|---|---|---|---|
| RLST0210 | 50 | -43 | 26 |
| RLST0212 | 51 | -41 | 32 |
| RLST0211 | 52 | -42 | 33 |
| RLST0157 | 57 | -40 | 40 |

A glass transition ($T_g$) and low melts ($T_{m1}$, $T_{m2}$) were observed in all the example polymers. The polymers all demonstrate a Tg around -40 °C corresponding to the amorphous soft segment. A gradual increase in Tm1 was observed as the amount of hard segment was increased.

It was observed that the melting peak was particularly broad for polymer RLST0208 (46% hard segment). The polyurethane block copolymers rarely fully phase separate but rather undergo liquid-liquid demixing. This phenomenon can make it difficult to clearly assign melting peaks other than attribute them to crystalline segments of telechelic diols and carbamate containing segments (hard segment).

**Gel Permeation Chromatography (GPC) analysis**

Molecular weight analysis of the example polymers was carried out by GPC (see Table 23).

Table 23: Molecular weights as determined by GPC

| sample | Mw (Da) | Mn (Da) | PDI |
|---|---|---|---|
| RLST0208-003 REX A | 57000 | 35400 | 1.60 |
| RLST0208-003 REX B | 57400 | 35600 | 1.61 |
| Mean | 57232 | 35537 | 1.61 |
| | | | |
| sample | Mw (Da) | Mn (Da) | PDI |
| RLST0210-001 REX A | 78200 | 48900 | 1.60 |
| RLST0210-001 REX B | 77900 | 48100 | 1.62 |
| Mean | 78000 | 48000 | 1.61 |
| | | | |
| sample | Mw (Da) | Mn (Da) | PDI |
| RLST211-001 A | 60500 | 41000 | 1.45 |
| RLST211-001 B | 62000 | 41900 | 1.48 |
| Mean | 61300 | 41800 | 1.47 |
| | | | |
| sample | Mw (Da | Mn (Da) | PDI |
| RLST212-001 A | N/A | N/A | N/A |
| RLST212-001 B | N/A | N/A | N/A |
| | | | |
| sample | Mw (Da) | Mn (Da) | PDI |
| RLST0213-001 REX A | 67300 | 42600 | 1.58 |
| RLST0213-001 REX B | 66500 | 42200 | 1.60 |
| Mean | 66900 | 42400 | 1.58 |

There were no significant differences in the polydispersity index (PDI) of the example polymers and the observed variation was within the expected 20% error margin. The GPC of elastomer RLST0212-001 was not run due to insufficient amount of sample.

**Wet granulation Formulations**

To improve the content uniformity of the quinagolide in the linear polymer and to facilitate further control over the initial burst release, a wet granulation formulation was developed (using RLST0210 as the base polymer). The formulation used excipients which bind with the drug and impede its release. Initially different binders such Zein, PVP K10 and ethyl cellulose were tested for their suitability. Due to their water soluble nature, Zein and PVP K 10 were discarded. However an ethyl cellulose based wet granulated formulation was found to be effective in minimising the burst release. Different ethyl cellulose concentrations were tested and an optimised level of 7% w/w was selected for future batches.

Although wet granulation significantly improved content uniformity it was found that due to electrostatic charges in the powder, the powder flow from these formulations was erratic. In order to rectify this problem fumed silica (Commercial name Aerosil®) was incorporated at 1.5% w/w. This improved flowability as well as the content uniformity of the final product.

The formulation details and mechanical data for all of the RLST0210 development batches tested are shown in Table 24 below and their release properties can be found in Table 25. It was found that a combination of the polymer and the quinagolide wet granulation formulation significantly reduces the quotient of the %24 hour release divided by the % drug released between 7 and 14 days for these formulations (0.24 -0.33).

Table 24: Formulation details and mechanical data for the RLST0210 development batches.

| Batch number | Formulation detail (wet granulation composition) | Mechanical Properties | | | |
|---|---|---|---|---|---|
| | | Elastic Modulus (MPa) | Load at Break (N) | Tensile Stress at Max Load (MPa) | Tensile Stress at 500% (%) |
| QH13058 | *Quinagolide HCl 0.05% w/w, Ethyl cellulose (EC) 7% w/w, Avicel 92.95% w/w | 29.77 | 148.68 | 14.59 | 7.46 |
| QH13059 | *Quinagolide HCl 0.12% w/w, Ethyl cellulose (EC) 7% w/w, Avicel 92.88% w/w | 27.33 | 171.54 | 15.02 | 7.32 |
| QH13060 | *Quinagolide HCl 0.5% w/w, Ethylcellulose (EC) 7% w/w, Avicel 92.50% w/w | 31.06 | 153.56 | 13.33 | 6.96 |
| QH13061 | *Quinagolide HCl 0.66% w/w, Ethyl cellulose (EC) 7% w/w, Avicel 92.34% w/w | 28.31 | 182.53 | 15.67 | 7.68 |
| QH13062 | *Quinagolide HCl 6.0% w/w, Ethyl cellulose (EC) 7% w/w, Avicel 87% w/w | 27.54 | 170.43 | 15.03 | 7.71 |
| QH13063 | *Quinagolide HCl 33.33% w/w, Ethyl cellulose (EC) 7% w/w, Avicel 59.67% w/w | 25.01 | 164.54 | 14.78 | 7.54 |
| QH13067R | *Quinagolide HCl 1.65% w/w, Ethyl cellulose (EC) 7% w/w, Avicel 91.35% w/w | 27.00 | 168.70 | 16.72 | 8.26 |
| QH13068R | *Quinagolide HCl 3.3% w/w, Ethyl cellulose (EC) 7% w/w, Avicel 89.70% w/w | 23.10 | 192.18 | 14.82 | 7.29 |
| QH13069R | *Quinagolide HCl 6.6% w/w, Ethyl cellulose (EC) 7% w/w, Avicel 86.40% w/w | 27.90 | 202.31 | 14.84 | 7.29 |

(continued)

| Batch number | Formulation detail (wet granulation composition) | Mechanical Properties | | | |
|---|---|---|---|---|---|
| | | Elastic Modulus (MPa) | Load at Break (N) | Tensile Stress at Max Load (MPa) | Tensile Stress at 500% (%) |
| QH14021R | *Quinagolide HCl 0.9% w/w, Ethylcellulose (EC) 7% w/w, Aerosil 1.5% w/w, Avicel 90.60% w/w | 24.01 | 193.21 | 14.72 | 6.08 |
| QH14022R | *Quinagolide HCl 1.8% w/w, Ethyl cellulose (EC) 7% w/w, Aerosil 1.5% w/w, Avicel 89.70% w/w | 25.03 | 209.41 | 15.74 | 5.97 |
| QH14023R | *Quinagolide HCl 2.7% w/w, Ethyl cellulose (EC) 7% w/w, Aerosil 1.5% w/w, Avicel 88.80% w/w | 23.34 | 192.09 | 13.98 | 6.27 |
| QH14024R | *Quinagolide HCl 0.833% w/w, Ethyl cellulose (EC) 7% w/w, Aerosil 1.5% w/w , Avicel 90.667% w/w | 24.92 | 179.37 | 15.65 | 5.93 |
| QH14025R | *Quinagolide HCl 1.667% w/w, Ethyl cellulose (EC) 7% w/w, Aerosil 1.5% w/w, Avicel 89.833% w/w | 24.46 | 180.78 | 15.80 | 6.18 |
| QH14028R | *Quinagolide HCl 2.5% w/w, Ethyl cellulose (EC) 7% w/w, Aerosil 1.5% w/w, Avicel 89.00% w/w | 24.65 | 237.28 | 17.95 | 5.97 |
| *In all batches IPA is used as a granulating solvent which was evaporated during the manufacturing process. | | | | | |

Table 25: Release data for the various RLST02010 formulations described in Table 24.

| | Formulation & Loading details | drug released in first 24h (%) | drug released between 7 and 14 day (%) | Quotient 24 h release/ 7-14 day release |
|---|---|---|---|---|
| QH13058 | 4mm Ring Units 0.02%w/w, 137μg/unit | 15.9 | * | * |
| QH13059 | 4mm Ring Units 0.10%w/w, 1008μg/unit | 3.6 | * | * |
| QH13060 | 4mm Ring Units 0.50%w/w, 4197μg/unit | 2.3 | * | * |
| QH13061 | 4mm Ring Units (minipig) 0.02%w/w, 218μg/unit | 27.4 | 19.5 | 0.33 |
| QH13062 | 4mm Ring Units (minipig) 0.18%w/w, 1689μg/unit | 6.2 | 10.9 | 0.28 |
| QH13063 | 4mm Ring Units (minipig) 1.00%w/w, 11362μg/unit | 1.5 | 2.6 | 0.28 |
| QH13067R | 4mm Ring Units (Sheep) 0.02%w/w, 400μg/unit | 14.3 | 25.0 | 0.24 |
| QH13068R | 4mm Ring Units (Sheep) 0.03%w/w, 800μg/unit | 14.4 | 27.1 | 0.24 |
| QH13069R | 4mm Ring Units (Sheep) 0.05%w/w, 1200μg/unit | 15.6 | 27.1 | 0.26 |

(continued)

| | Formulation & Loading details | drug released in first 24h (%) | drug released between 7 and 14 day (%) | Quotient 24 h release/ 7-14 day release |
|---|---|---|---|---|
| QH13070R | 4mm Ring Units 0.07%w/w, 1550μg/unit | 12.1 | 18.7 | 0.29 |
| QH13071R | 4mm Ring Units 0.10% w/w, 2450μg/unit | 5.3 | 10.3 | 0.26 |
| QH13072R | 4mm Ring Units 0.10%w/w, 2500μg/unit | 4.9 | 8.9 | 0.27 |
| QH13073X | Extruded Rods (REX) 0.01 %w/w, 200μg/unit | 8.5 | * | * |
| QH13074M | Melt Mix Blocks 0.03%w/w, 400μg/unit | 3.6 | 3.0 | 0.33 |
| QH14021R | 4mm Ring Units (CTA) 0.02%w/w, 400μg/unit | 5.8 | 9.1 | 0.29 |
| QH14022R | 4mm Ring Units (CTA) 0.03%w/w, 800μg/unit | 4.5 | 7.4 | 0.24 |
| QH14023R | 4mm Ring Units (CTA) 0.04%w/w, 1200μg/unit | 4.3 | 8.6 | 0.26 |
| QH14024R | 4mm Ring Units (Phase I) 0.02%w/w, 400μg/unit | 5.3 | 7.5 | 0.29 |
| QH14025R | 4mm Ring Units (Phase I) 0.03%w/w, 800μg/unit | 4.4 | 8.7 | 0.25 |
| QH14028R | 4mm Ring Units (Phase I) 0.04%w/w, 1200μg/unit | 4.6 | 8.5 | 0.26 |

To provide an indication of the mechanical properties of the rings *in vivo,* batches QH13067R, QH13068R and QH13069R were also assessed after being hydrated for a period of 48 hours. The results are illustrated in Table 26 below.

Table 26: Mechanical properties after 48hrs hydration.

| Batch number | Formulation detail (wet granulation composition) | Mechanical Properties | | | |
|---|---|---|---|---|---|
| | | Elastic Modulus (MPa) | Load at Break (N) | Tensile Stress at Max Load (MPa) | Tensile Stress at 500% (%) |
| QH13067R | *Quinagolide HCl 1.65% w/w Ethyl cellulose (EC) 7% w/w Avicel 91.35% w/w | 13.73 | 143.37 | 11.99 | 5.57 |
| QH13068R | *Quinagolide HCl 3.3% w/w Ethylcellulose (EC) 7% w/w Avicel 89.70% w/w | 14.26 | 118.03 | 11.43 | 5.49 |
| QH13069R | *Quinagolide HCl 6.6% w/w Ethyl cellulose (EC) 7% w/w Avicel 86.40% w/w | 16.29 | 136.91 | 11.09 | 5.46 |

It was observed that after hydration, the elastic modulus of RLST0210 is reduced to around 13-16 MPa. Therefore, after hydration this polymer has an elastic modulus comparable to the elastic modulus of the commercially available Nuvaring® product.

## Claims

1. A polymeric drug-device unit comprising:

   (i) a polyurethane block copolymer obtainable by reacting together:

      (a) a poly(alkylene oxide);
      (b) a difunctional compound;
      (c) a difunctional isocyanate; and
      (d) optionally a block copolymer comprising poly(alkylene oxide) blocks; and

   (ii) quinagolide or a pharmaceutically acceptable salt thereof, as a pharmaceutically active agent.

2. The polymeric drug-device unit of claim 1, wherein the poly(alkylene oxide) is a polyethylene glycol (PEG) or a polypropylene glycol (PPG).

3. The polymeric drug-device unit of any one of claims 2, wherein the polypropylene glycol has a molecular weight of 200 to 35,000 g/mol or approximately 2,000 g/mol.

4. The polymeric drug-device unit of any one of claim 2, wherein the polyethylene glycol has a molecular weight of 200 to 35,000 g/mol or a molecular weight of approximately 2,000 g/mol.

5. The polymeric drug-device unit of any preceding claim, wherein the poly(alkylene oxide) block copolymer comprises blocks of polyethylene glycol and polypropylene glycol.

6. The polymeric drug-device unit of any preceding claim, wherein the difunctional compound is selected from the group consisting of diols; diamines; and amino alcohols; optionally, wherein the diol is a $C_3$ to $C_{20}$ diol or the difunctional compound is selected from the group consisting of: 1,4-butanediol; 1,5-pentanediol; 1,6-hexanediol; 1,10-decanediol; 1,12-dodecanediol; and 1,16-hexadecanediol.

7. The polymeric drug-device unit of any preceding claim, wherein the difunctional isocyanate is an aromatic diisocyanate or an aliphatic diisocyanate; optionally, wherein the difunctional isocyanate is diphenylmethane-4,4'-diisocyanate, dicyclohexylmethane-4,4'-diisocyanate (DMDI) or hexamethylene diisocyanate (HMDI).

8. The polymeric drug-device unit of any preceding claim, wherein the molar ratio of the components (a) to (b) to (c) is in the range 0.05-0.75 to 1 to 1.00-2.00.

9. The polymeric drug-device unit of any preceding claim, wherein the ratio of components (a) to (b) to (c) to (d) is in the range 0.05-0.75 to 1 to 1.00-2.00 to 0.01-0.50.

10. The polymeric drug-device unit of any preceding claim, wherein the device is obtainable by reacting together components (a), (b), (c) and optionally (d) in the presence of a catalyst; optionally, wherein the catalyst is a ferric chloride and/or bismuth based catalysts.

11. The polymeric drug-device unit of any preceding claim, wherein the polymeric drug-device unit comprises one or more polyurethane block copolymers, wherein the, or each, polyurethane block copolymer is obtainable by reacting together:

    (a) a poly(alkylene oxide);
    (b) a difunctional compound;
    (c) a difunctional isocyanate; and
    (d) optionally a block copolymer comprising poly(alkylene oxide) blocks.

12. The polymeric drug-device unit of claim 11, wherein the polymeric drug-device unit comprises a monolithic-type or single matrix-type polymer structure; a reservoir structure; a layered structure, each layer comprising one or more of the polyurethane block copolymers; or an inner core structure or layer and an outer layer, cap, sheath, or coating.

13. The polymeric drug-device unit of claim 12, wherein the inner core structure or layer is loaded with quinagolide.

**14.** The polymeric drug-device unit of claim 12, wherein quinagolide is absent from the outer layer or coating.

**15.** The polymeric drug-device unit of any preceding claim, wherein in use, the initial quinagolide release conforms to a release quotient of between 0.05 and 10, the release quotient being calculated as the percentage release over an initial 24 hour period divided by the percentage of quinagolide release over a later period; optionally wherein the percentage of quinagolide release over a later period is the percentage of quinagolide release over the period of 7-14 days after administration.

**16.** The polymeric drug-device unit of any preceding claim, wherein the polymeric drug-device unit comprises a resilient, deformable/flexible and/or soft polymer.

**17.** The polymeric drug-device unit of any preceding claim, wherein the polymeric drug-device unit takes the form of a ring for insertion and/or location into the vaginal cavity.

**18.** The polymeric drug-device unit of any preceding claim, wherein the polymeric drug-device unit has an elastic modulus between about 5 and 100 MPa; optionally wherein the elastic modulus between about 5 and 30 MPa, between 10 and 20 MPa or between about 10 and 20 MPa when in a hydrated state.

**19.** The polymeric drug-device unit of any preceding claim, wherein the polymeric drug-device unit comprises quinagolide or a pharmaceutically acceptable salt thereof at a dose of about 25 to about 15000 micrograms μg; optionally wherein the polymeric drug-device unit comprises quinagolide or a pharmaceutically acceptable salt thereof at a dose of about 200 to 5000 μg, about 400-1500 μg, about 200 μg, about 400 μg, about 800 μg or about 1200 μg quinagolide.

**20.** The polymeric drug-device unit of any preceding claim, wherein the polymeric drug-device unit provides a continuous release of quinagolide to the vaginal tissues; optionally, wherein the polymeric drug-device unit continuously releases quinagolide over a period of about 21, 28 or 35 days.

**21.** The polymeric drug-device unit of any preceding claim, wherein in use, the polymeric drug-device unit releases between about 1 and about 50 μg quinagolide/day; optionally,
wherein the polymeric drug-device unit releases about 5, about 10, about 15, about 20 or about 30 μg quinagolide/day.

**22.** The polymeric drug-device unit of any preceding claim, wherein the quinagolide or a pharmaceutically acceptable salt thereof, is loaded into the polymeric drug-device unit as a granulated formulation, e.g. a wet granulated formulation.

**23.** The polymeric drug-device unit of any preceding claim, wherein the quinagolide or a pharmaceutically acceptable salt thereof is formulated with one or more excipients; optionally,
wherein the excipients are selected from the group consisting of cellulose (e.g. microcrystalline cellulose), cellulose derivatives (such as ethyl cellulose, (hydroxypropyl)methyl cellulose (HPMC) and hydroxypropyl cellulose (HPC)), polysaccharides (such as pregelatinised starch and pullulan), Zein and polyvinylpyrrolidone (PVP).

**24.** The polymeric drug-device unit of any preceding claim, wherein the quinagolide or a pharmaceutically acceptable salt thereof, is loaded into the device using an antistatic additive; optionally, wherein the antistatic additive is fumed silica.

**25.** A method of making a polymeric drug-device unit for the intravaginal administration of quinagolide or a pharmaceutically acceptable salt thereof, said method comprising reacting together:

(a) a poly(alkylene oxide);
(b) a difunctional compound;
(c) a difunctional isocyanate; and
(d) optionally a block copolymer comprising poly(alkylene oxide) blocks;
to provide a polyurethane block copolymer; and loading quinagolide or a pharmaceutically acceptable salt thereof into the polyurethane block copolymer.

**26.** The method of claim 25, wherein the polyurethane block copolymer is prepared by a reactive extrusion process or a batch process.

**27.** The method of claim 25 or 26, wherein the quinagolide or a pharmaceutically acceptable salt thereof, is loaded into the polyurethane block copolymer via a hot melt extrusion process.

**28.** The method of any one of claim 25, 26 or 27, wherein the quinagolide is formulated into granules before loading.

**29.** A polymeric drug-device unit according to any one of claim 1-24 or prepared by a method according to any one of claims 25-28, for use in the treatment and/or prevention of endometriosis.

**30.** A method of treating and/or preventing endometriosis, said method comprising administering to a subject in need thereof, a polymeric drug-device unit according to any one of claim 1-24 or prepared by a method according to any one of claims 25-28.

**31.** A kit comprising a polymeric drug-device unit according to any one of claim 1-24 or prepared by a method according to any one of claims 25-28; optionally, wherein the kit further comprises an applicator and/or instructions for use.

**32.** The kit of claim 31, wherein the applicator facilitates insertion of the polymeric drug-device unit into a vaginal cavity; optionally wherein the polymeric drug-device unit is pre-loaded into or onto the applicator.

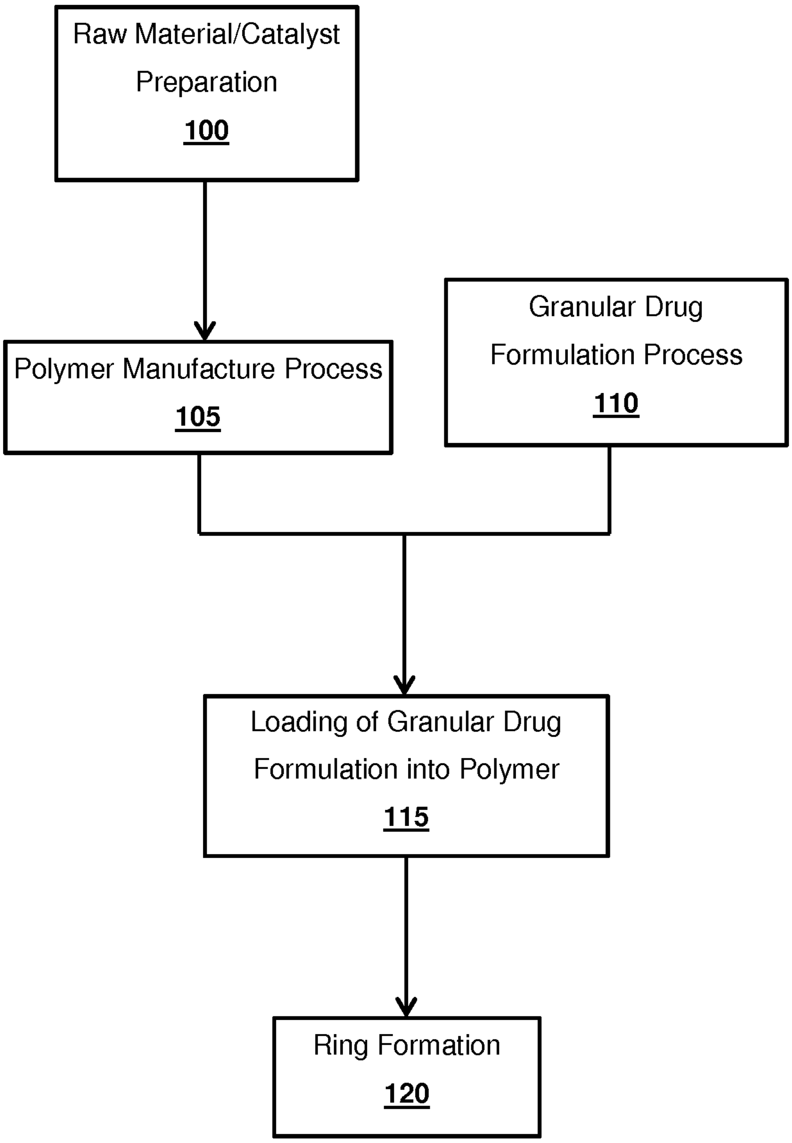
Raw Material/Catalyst Preparation
100
Polymer Manufacture Process
105
Granular Drug Formulation Process
110
Loading of Granular Drug Formulation into Polymer
115
Ring Formation
120

**Figure 1**

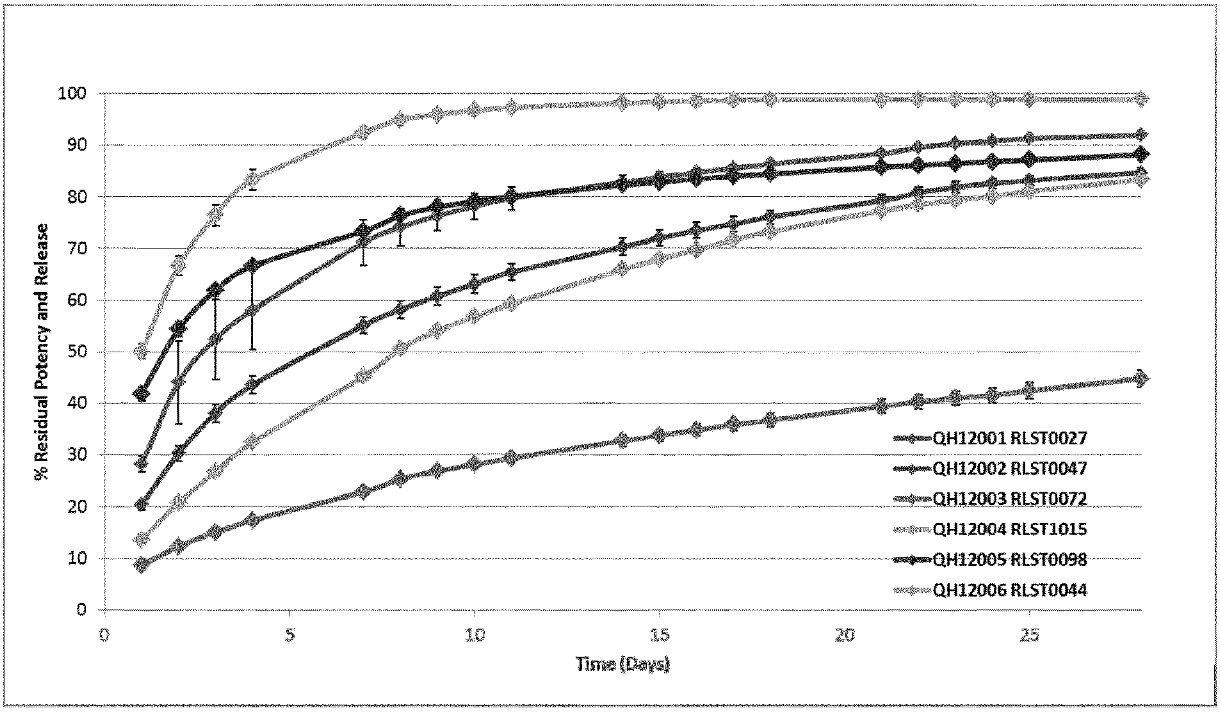
100
90
80
70
60
50
40
30
20
10
0
% Residual Potency and Release
0
5
10
15
20
25
Time (Days)
QH12001 RLST0027
QH12002 RLST0047
QH12003 RLST0072
QH12004 RLST1015
QH12005 RLST0098
QH12006 RLST0044

Figure 2

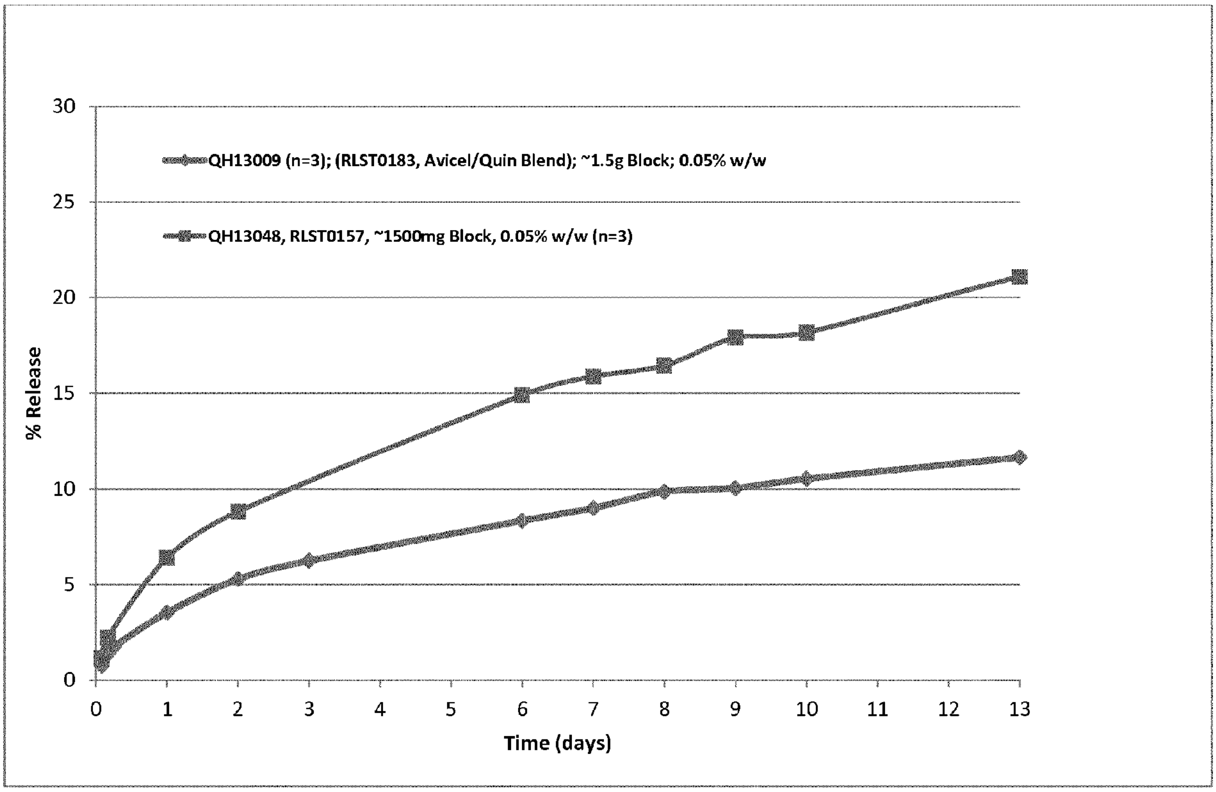
QH13009 (n=3); (RLST0183, Avicel/Quin Blend); ~1.5g Block; 0.05% w/w
QH13048, RLST0157, ~1500mg Block, 0.05% w/w (n=3)
% Release
30
25
20
15
10
5
0
0
1
2
3
4
5
6
7
8
9
10
11
12
13
Time (days)

Figure 3

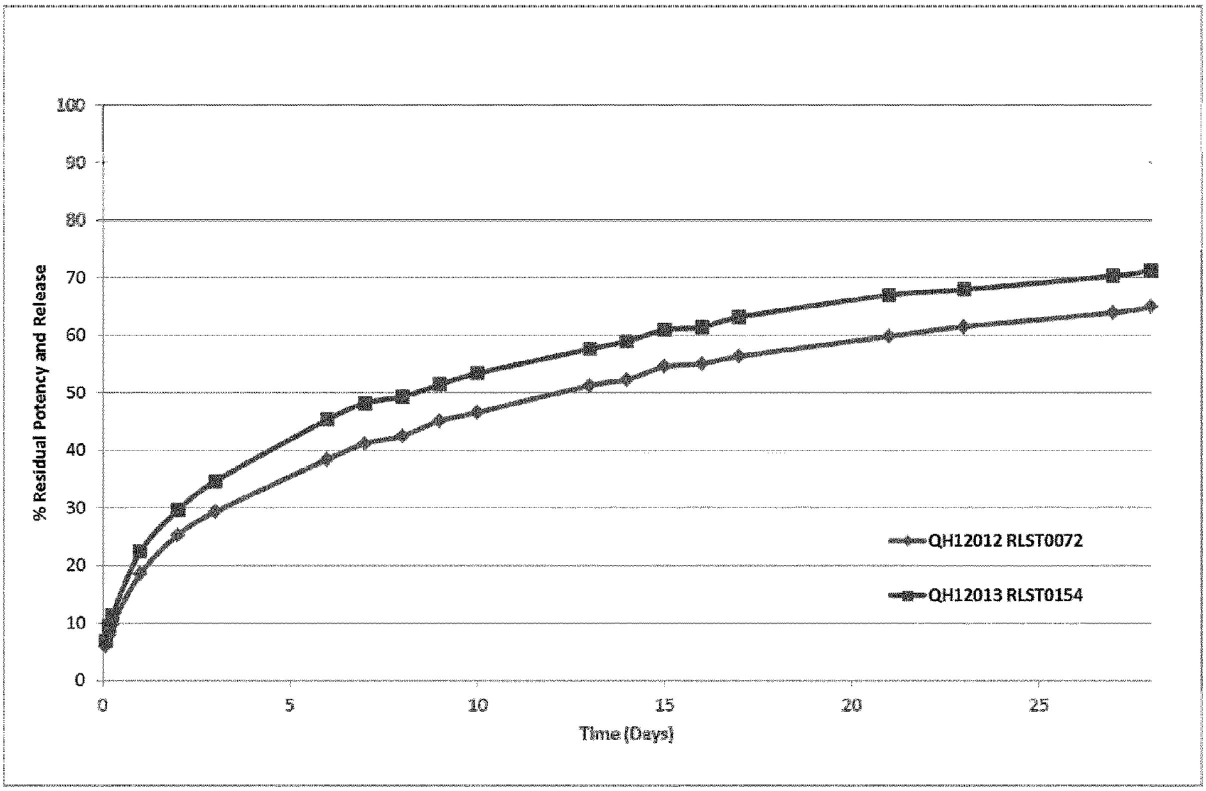
100
90
80
70
60
50
40
30
20
10
0
% Residual Potency and Release
0
5
10
15
20
25
Time (Days)
QH12012 RLST0072
QH12013 RLST0154

Figure 4

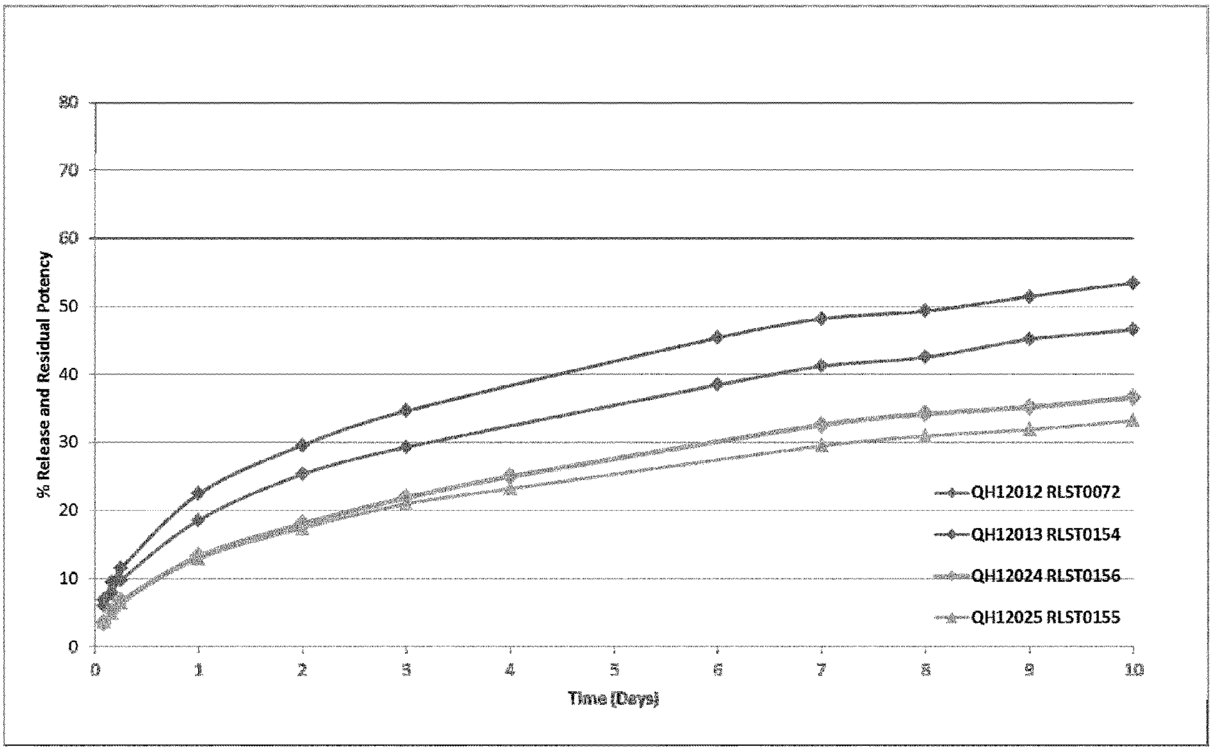
% Release and Residual Potency
80
70
60
50
40
30
20
10
0
0
1
2
3
4
5
6
7
8
9
10
Time (Days)
QH12012 RLST0072
QH12013 RLST0154
QH12024 RLST0156
QH12025 RLST0155

Figure 5

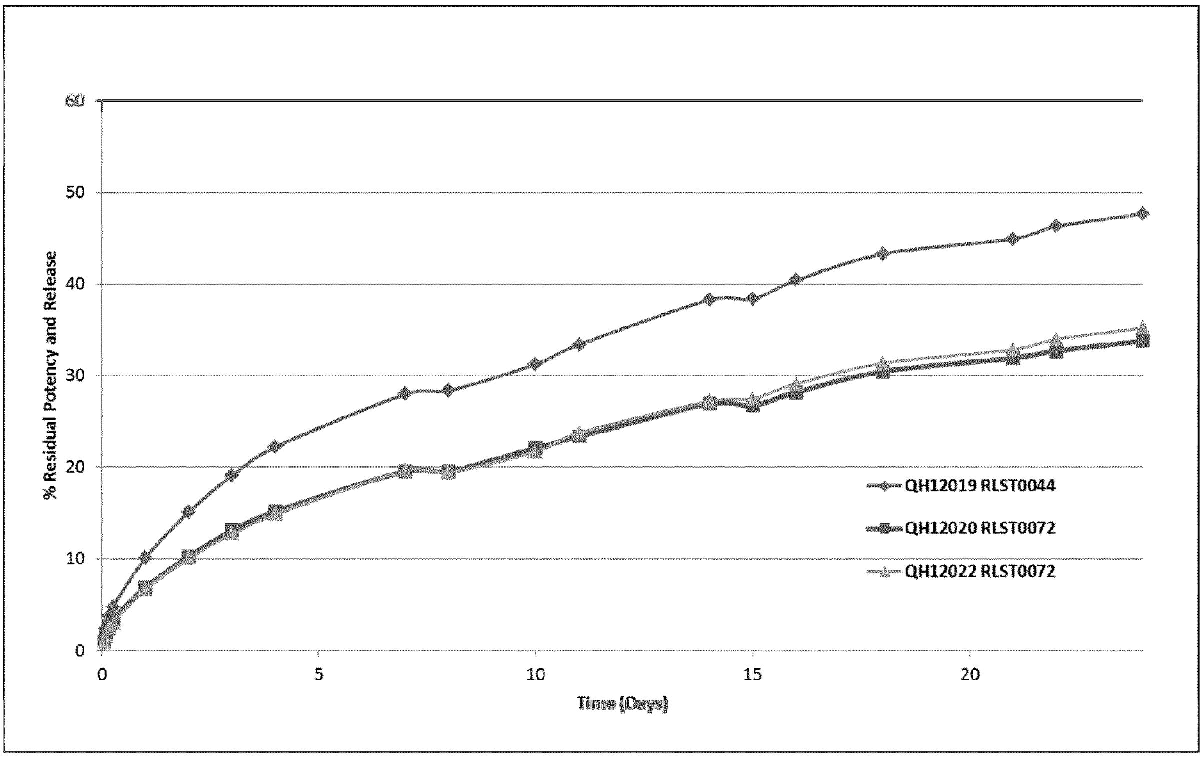
% Residual Potency and Release
60
50
40
30
20
10
0
0
5
10
15
20
Time (Days)
QH12019 RLST0044
QH12020 RLST0072
QH12022 RLST0072

Figure 6

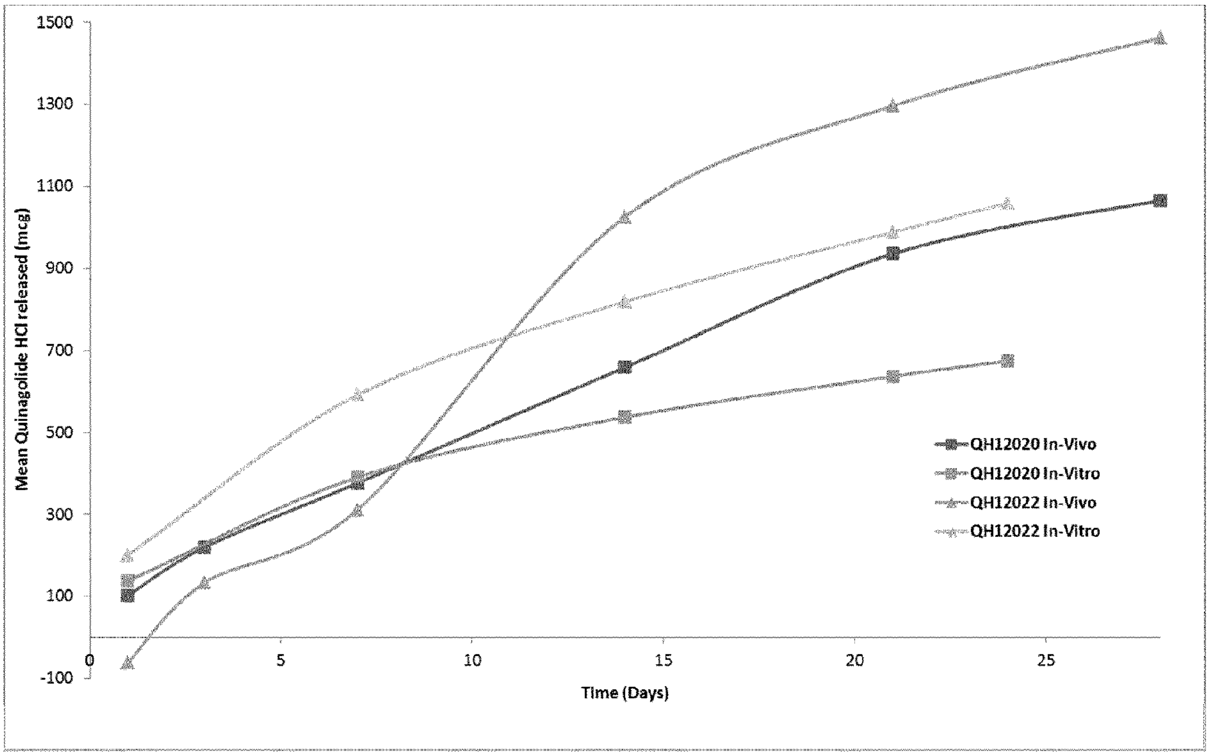

1500
1300
1100
900
700
500
300
100
-100
0
5
10
15
20
25
Mean Quinagolide HCl released (mcg)
Time (Days)
QH12020 In-Vivo
QH12020 In-Vitro
QH12022 In-Vivo
QH12022 In-Vitro


Figure 7

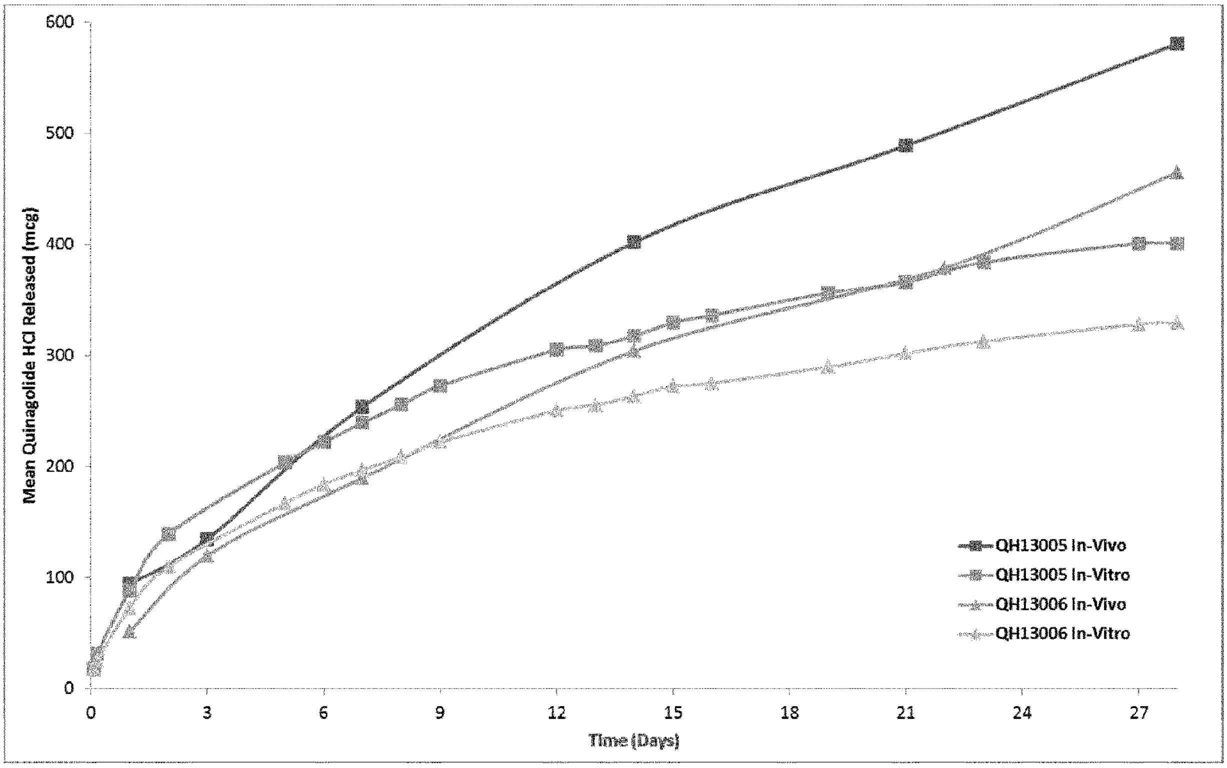
Mean Quinagolide HCl Released (mcg)
600
500
400
300
200
100
0
0
3
6
9
12
15
18
21
24
27
Time (Days)
QH13005 In-Vivo
QH13005 In-Vitro
QH13006 In-Vivo
QH13006 In-Vitro

Figure 8

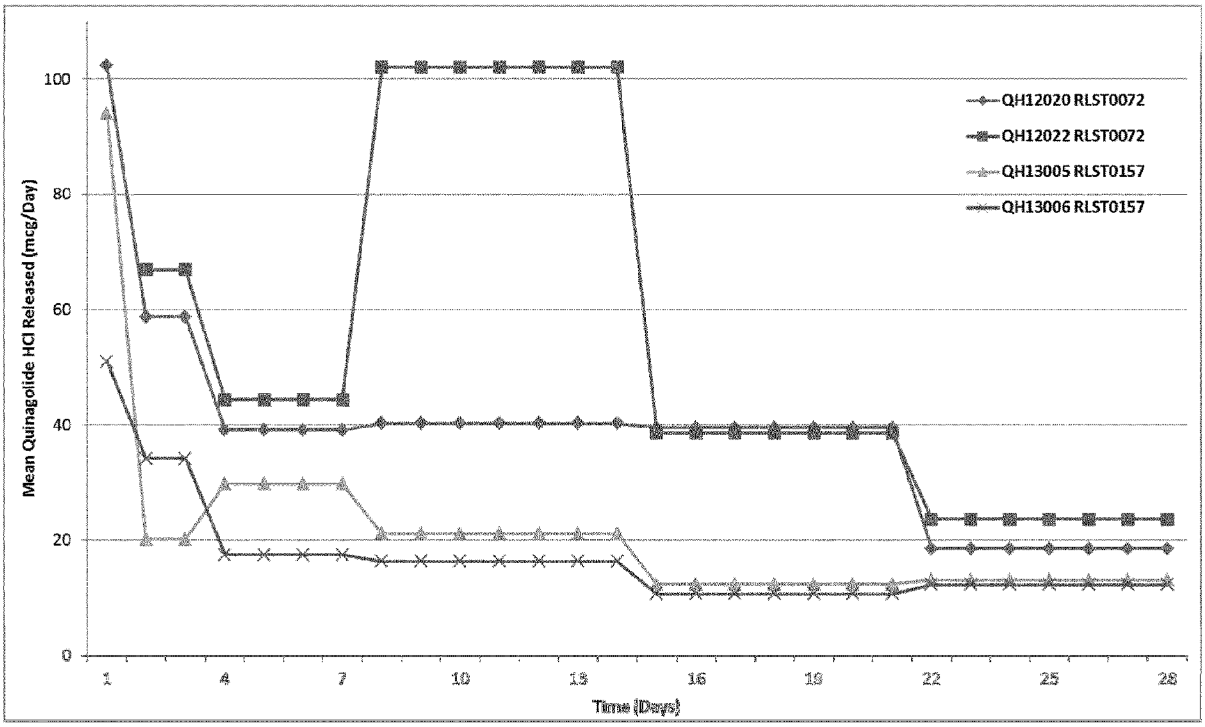
Mean Quinagolide HCl Released (mcg/Day)
Time (Days)
0
20
40
60
80
100
1
4
7
10
13
16
19
22
25
28
QH12020 RLST0072
QH12022 RLST0072
QH13005 RLST0157
QH13006 RLST0157

Figure 9

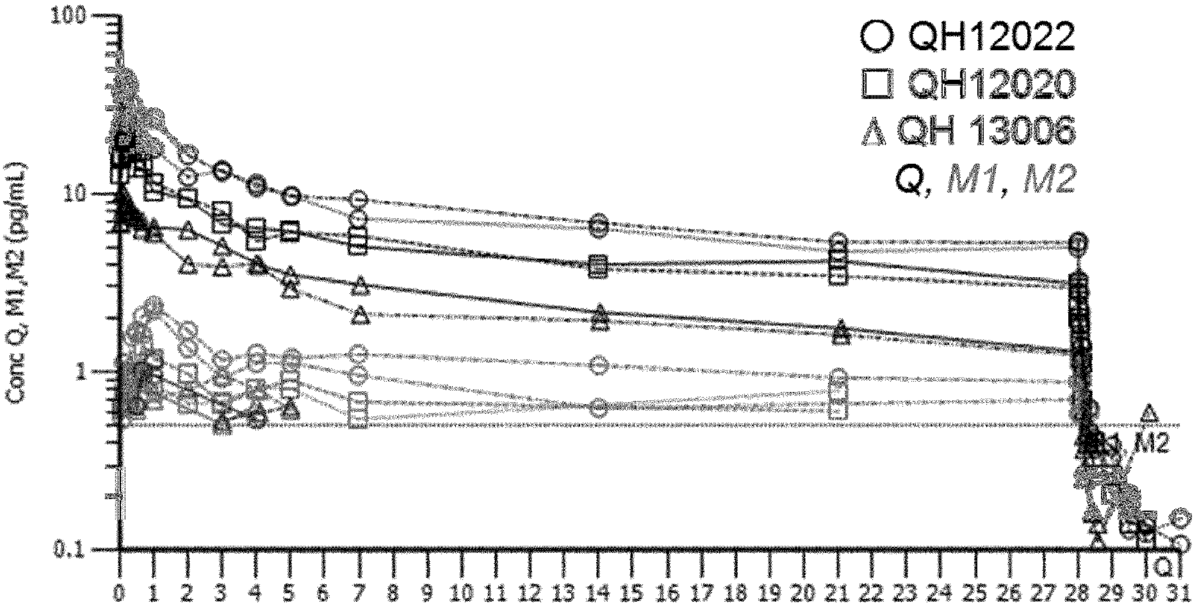

100
10
1
0.1
Conc Q, M1,M2 (pg/mL)
O QH12022
□ QH12020
Δ QH 13006
Q, M1, M2
M1, M2
Q
0 1 2 3 4 5 6 7 8 9 10 11 12 13 14 15 16 17 18 19 20 21 22 23 24 25 26 27 28 29 30 31


Figure 10

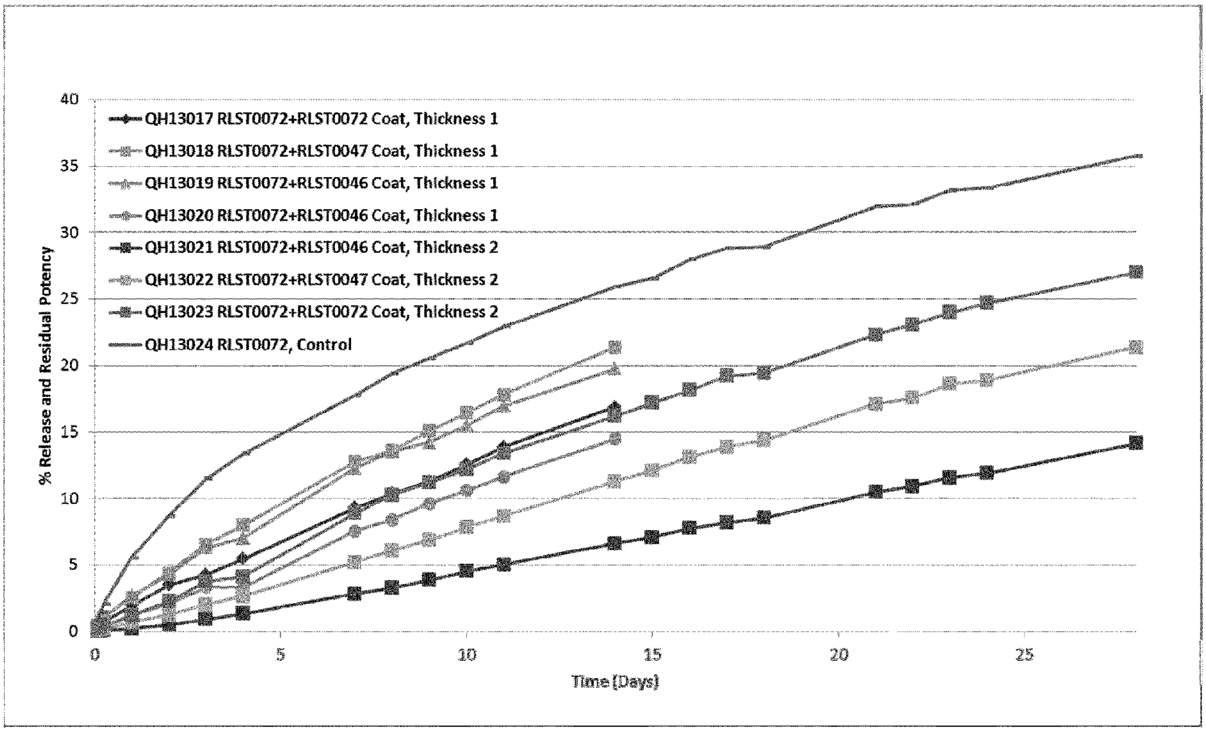
QH13017 RLST0072+RLST0072 Coat, Thickness 1
QH13018 RLST0072+RLST0047 Coat, Thickness 1
QH13019 RLST0072+RLST0046 Coat, Thickness 1
QH13020 RLST0072+RLST0046 Coat, Thickness 1
QH13021 RLST0072+RLST0046 Coat, Thickness 2
QH13022 RLST0072+RLST0047 Coat, Thickness 2
QH13023 RLST0072+RLST0072 Coat, Thickness 2
QH13024 RLST0072, Control
% Release and Residual Potency
Time (Days)
0
5
10
15
20
25
30
35
40

Figure 11

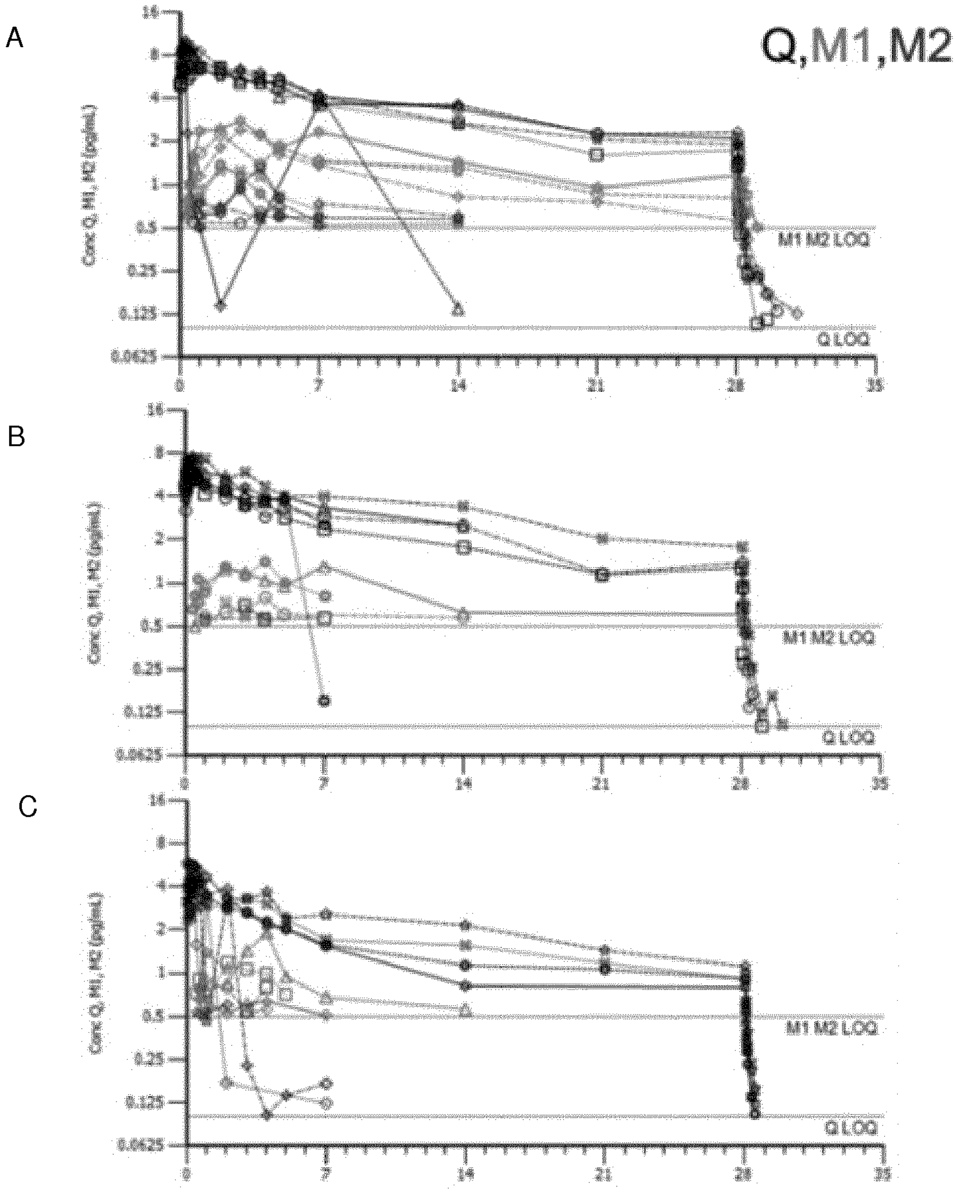


Figure 12 A, B and C

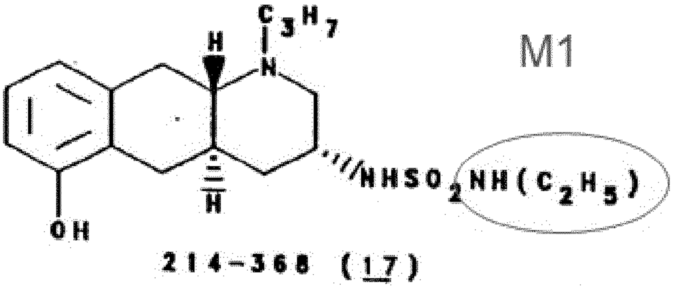

M1

214-368 (17)

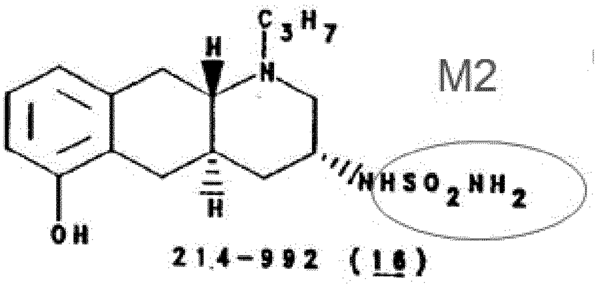

M2

214-992 (16)

Figure 13

Europäisches Patentamt
European Patent Office
Office européen des brevets

# EUROPEAN SEARCH REPORT

Application Number
EP 14 19 2372

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2008/007046 A1 (CONTROLLED THERAPEUTICS SCT [GB]; TUOMINEN JUKKA [GB]; ZURUTUZA AMAIA) 17 January 2008 (2008-01-17)<br>* page 9, lines 12-17; claims 1,14; example 1; table 1 *<br>----- | 1-32 | INV.<br>A61K9/00<br>A61K47/34<br>A61K31/00 |
| Y,D | WO 2013/013172 A1 (UNIV UTAH RES FOUND [US]; KISER PATRICK F [US]; JOHNSON TODD JOSEPH [U) 24 January 2013 (2013-01-24)<br>* paragraphs [0001], [0004]; claims 1, 10, 24, 44 *<br>----- | 1-32 | |
| Y | WO 2010/150098 A2 (FERRING INT CT SA [CH]; PELLICER-MARTINEZ ANTONIO [ES]; SIMON-VALLES C)<br>29 December 2010 (2010-12-29)<br>* page 1, lines 20-24 *<br>* page 8, lines 2-4 *<br>----- | 1-32 | |

| TECHNICAL FIELDS SEARCHED (IPC) |
|---|
| A61K |

The present search report has been drawn up for all claims

1

| Place of search | Date of completion of the search | Examiner |
|---|---|---|
| The Hague | 27 March 2015 | Schwald, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 19 2372

This annex lists the patent family members relating to the patent documents cited in the above-mentioned European search report. The members are as contained in the European Patent Office EDP file on
The European Patent Office is in no way liable for these particulars which are merely given for the purpose of information.

27-03-2015

| Patent document cited in search report | Publication date | Patent family member(s) | Publication date |
|---|---|---|---|
| WO 2008007046 A1 | 17-01-2008 | AT 498643 T | 15-03-2011 |
| | | AU 2007274119 A1 | 17-01-2008 |
| | | BR PI0713481 A2 | 06-11-2012 |
| | | CA 2656788 A1 | 17-01-2008 |
| | | CN 101484495 A | 15-07-2009 |
| | | DK 2038325 T3 | 30-05-2011 |
| | | EP 2038325 A1 | 25-03-2009 |
| | | ES 2363704 T3 | 12-08-2011 |
| | | JP 5307710 B2 | 02-10-2013 |
| | | JP 5639673 B2 | 10-12-2014 |
| | | JP 2009542831 A | 03-12-2009 |
| | | JP 2013151517 A | 08-08-2013 |
| | | PT 2038325 E | 02-05-2011 |
| | | US 2009324692 A1 | 31-12-2009 |
| | | US 2012329883 A1 | 27-12-2012 |
| | | WO 2008007046 A1 | 17-01-2008 |
| WO 2013013172 A1 | 24-01-2013 | AU 2012283837 A1 | 27-02-2014 |
| | | CA 2842550 A1 | 24-01-2013 |
| | | CN 103796708 A | 14-05-2014 |
| | | EP 2734262 A1 | 28-05-2014 |
| | | JP 2014520896 A | 25-08-2014 |
| | | KR 20140074281 A | 17-06-2014 |
| | | US 2014209100 A1 | 31-07-2014 |
| | | WO 2013013172 A1 | 24-01-2013 |
| WO 2010150098 A2 | 29-12-2010 | AR 080541 A1 | 18-04-2012 |
| | | CA 2765997 A1 | 29-12-2010 |
| | | EP 2445501 A2 | 02-05-2012 |
| | | JP 2012530776 A | 06-12-2012 |
| | | TW 201102066 A | 16-01-2011 |
| | | US 2012157489 A1 | 21-06-2012 |
| | | US 2014194460 A1 | 10-07-2014 |
| | | WO 2010150098 A2 | 29-12-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20120157489 A
- US 20100113499 A
- US 20080293693 A
- WO 2009094573 A
- WO 2010019226 A
- WO 2004096151 A
- US 4235988 A
- WO 2005004837 A
- WO 2013013172 A
- WO 2008007046 A
- WO 2012066000 A


### Non-patent literature cited in the description


- *Eur. J. Endocrinol,* 01 February 2006, vol. 154, 187-195
- **M.R. CLARK et al.** *Journal of Pharmaceutical Sciences,* February 2012, vol. 101 (2